# EUROPÄISCHE PATENTSCHRIFT

(11) **EP 4 021 483 B1**
(45) Veröffentlichungstag und Bekanntmachung des Hinweises auf die Patenterteilung: **25.09.2024**
(21) Anmeldenummer: 20793290.6
(22) Anmeldetag: 12.10.2020
(51) Int. Cl.: A61K 38/48, A61K 51/00, A61P 5/00, A61P 35/00, C07K 14/33, A61K 31/00, A61K 31/46, A61K 51/04, A61P 39/00

(54) **BOTULINUMTOXIN IN EINEM VERFAHREN ZUR PRÄVENTION VON STRAHLENSCHÄDEN IN HUMANEN DRÜSEN**
BOTULINUMTOXIN FOR USE IN A METHOD FOR PREVENTING HUMAN GLANDS FROM RADIATION DAMAGE
BOTULINUMTOXIN POUR UTILISATION DANS UNE MÉTHODE POUR EMPÊCHER LES GLANDES HUMAINES DE SUBIR DES DOMMAGES DUS AU RAYONNEMENT

(30) Priorität: 10.10.2019 DE 102019215585
(43) Veröffentlichungstag der Anmeldung: 06.07.2022
(73) Patentinhaber: Merz Therapeutics GmbH, 60318 Frankfurt am Main (DE)
(72) Erfinder: MÜLLER, Jörg, 14197 Berlin (DE); BAUM, Richard, 55595 Hargesheim (DE)
(74) Vertreter: Grund, Martin
(86) Internationale Anmeldenummer: PCT/EP2020/078619
(87) Internationale Veröffentlichungsnummer: WO 2021/069740

(56) Entgegenhaltungen:
- WO-A1-2016/014750
- WO-A2-2012/103038
- DE-T2- 60 214 134
- US-A- 6 139 845
- US-A1- 2008 063 732
- US-A1- 2018 311 499
- BAUM RICHARD P ET AL: "Injection of Botulinum Toxin for Preventing Salivary Gland Toxicity after PSMA Radioligand Therapy: an Empirical Proof of a Promising Concept", NUCLEAR MEDICINE AND MOLECULAR IMAGING, SPRINGER BERLIN HEIDELBERG, BERLIN/HEIDELBERG, vol. 52, no. 1, 11 January 2018 (2018-01-11), pages 80 - 81, XP036414256, ISSN: 1869-3474, [retrieved on 20180111], DOI: 10.1007/S13139-017-0508-3

## Beschreibung

### Gebiet der Erfindung

Die vorliegende Erfindung liegt auf dem Gebiet der Radioonkologie. Unter Verwendung von Botulinumtoxin und Anticholinergikum können Strahlenschäden durch Radioliganden in humanen Drüsen vermieden werden. Durch ein optimiertes Therapieschema kann dem Verfahren weitere vorteilhafte Eigenschaften verliehen werden. Hierbei werden sowohl bildgebende Verfahren zur Diagnostik als auch Therapieverfahren umfasst.

Ebenfalls offenbart die Erfindung die Verwendung von Botulinumtoxin Typ B zur Prävention von Strahlenschäden der Ohrspeichel- und Unterkieferspeicheldrüsen, sowie die Verwendung von Botulinumtoxin Typ A zur Prävention von Strahlenschäden der Drüsen.

### Hintergrund der Erfindung

Ein Radioligand ist eine mit einem Radionuklid markierte Substanz, die als ein Ligand an ein Zielprotein, beispielsweise einen Rezeptor, binden kann. Solche Radioliganden werden beispielsweise sowohl in der Diagnostik als auch in der Therapie von onkologischen Erkrankungen eingesetzt. Ein prominentes Beispiel sind PSMA-Radionuklide in der Diagnostik und Behandlung des Prostatakarzinoms.

PSMA (Prostataspezifisches Membranprotein) ist ein Transmembranprotein, das von Prostatakarzinomzellen bis zu 1000-fach stärker exprimiert wird als von normalen Prostatazellen. PSMA ist deshalb ein ideales Zielprotein für die Diagnostik und zugleich auch für die zielgerichtete Therapie von Prostatakrebs. PSMA wird auch von anderen Karzinomen exprimiert, obgleich in geringerer Konzentration im Vergleich zum Prostatakarzinom. PSMA wird ebenso von Speicheldrüsen und auch Augendrüsen exprimiert. Eine bleibende Schädigung der genannten Drüsen führt zu schwerster Mundtrockenheit mit mannigfaltigen Komplikationen wie Zahnschäden und chronischer Augentrockenheit, die zu Sehstörungen führen kann.

PSMA-Radionuklide werden sowohl diagnostisch, als auch therapeutisch eingesetzt.

Die PSMA-Radioliganden ⁶⁸Ga-PSMA und ¹⁸F-PSMA werden zur Diagnostik des primären und Rezidiv-Prostatakarzinoms und der Suche nach Metastasen eingesetzt. Die diagnostischen Liganden reichern sich in den Prostatakarzinomzellen und den anderen oben genannten Organen/Tumoren an, die diagnostischen PSMA-Radioliganden können Augen- und Speicheldrüsen schädigen.

Die therapeutisch eingesetzten PSMA-Radioliganden verursachen immer eine je nach Radioligand unterschiedlich ausgeprägte und häufig persistierende Speicheldrüsenschädigung mit erheblichen gesundheitlichen Problemen und Einschränkung der Lebensqualität (Mundtrockenheit, Ulcera im gesamten Mundbereich, Schluckstörungen, Aspirationsgefahr, Zahnschäden etc.). Problematisch ist hierbei auch, dass die PSMA-Radiologanden häufig wiederholt verabreicht werden, so dass die Drüsenschädigung akkumuliert. PSMA-Radioliganden verursachen auch eine je nach Radioligand unterschiedlich ausgeprägte Augendrüsenschädigung mit gesundheitlichen Folgen: trockene Augen, wiederholte Augenentzündungen, chronische Augenentzündungen und Sehstörungen. Diese Schädigung durch die PSMA-Therapie ist häufig irreversibel. Ferner führen diese Schädigungen und Komplikationen oft dazu, dass Prostatakarzinompatienten die lebensverlängernde Therapie nicht weiter wahrnehmen können.

Vermutlich besitzen unterschiedliche PSMA-Radioliganden eine unterschiedliche Affinität zu den einzelnen Speicheldrüsen und somit eine unterschiedliche Toxizität. Bei den verschiedenen Radionukliden handelt es sich entweder um Alpha- oder Beta- oder Gammastrahler. Aktuell werden beispielsweise die Nuklide ¹⁷⁷Lu (ein Betastrahler mit einer Reichweite von 2 mm) und 225Ac (ein Alphastrahler mit einer Reichweite von 50-90 µm) eingesetzt. Zukünftig sind noch wirksamere, auf sehr kurze Distanz (50-90 µm) stark strahlende PSMA-Radioliganden für den therapeutischen Einsatz zu erwarten. Die Hinweise verdichten sich, dass ²²⁵Ac die Speicheldrüsen mehr beschädigt als ¹⁷⁷Lu.

Durch den Einsatz von noch wirksameren Radioliganden, wie beispielsweise Alphastrahlern, sind Drüsen einem noch höheren Risiko ausgesetzt, beschädigt zu werden.

Drüsen nehmen vielfältige Aufgaben im menschlichen Körper war. Es wird unter Anderem zwischen endokrinen und exokrinen Drüsen unterschieden. Falls ein Target nicht nur beispielsweise in einem Tumor exprimiert wird, sondern auch in Drüsen wie zum Beispiel die PSMA-Expression in Speicheldrüsen und auch Augendrüsen, werden auch die gesunden Drüsen angegriffen.

An dieser Stelle kann Botulinumtoxin Abhilfe schaffen: Botulinumtoxin-Injektionen können im Rahmen einer abgestimmten Methode die Drüsen schützen. Botulinumtoxin ist ein generischer Begriff für die Familie von Toxinen, die durch das Bakterium *Clostridium botulinum* hergestellt werden, darstellt. Aktuell sind sieben unterschiedliche Botulinumtoxine (A, B, C, D, E, F und G) bekannt, die sich in ihren Eigenschaften voneinander unterscheiden. Auch die Wirkmechanismen der verschiedenen Botulinumtoxin Typen unterscheidet sich: beispielsweise beruht die Hauptwirkung von Typ A auf der Spaltung von intrazellulärem SNAP-25, während Typ B hauptsächlich das "vehicle associated membrane protein" (=VAMP) spaltet. Auch verfügt Botulinumtoxin Typ B im Vergleich mit Typ A über weitere Unterschiede: Typ B bindet präsynaptisch an das Syt-II-Protein, ebenso präsynaptisch besteht eine partielle Interaktion zwischen Typ B und Tetanustoxin-Residuen und eine Neurotoxin-Gangliosid-Interaktion mit den Gangliosiden GD1a, GD1b und GM1a . Lokale Botulinumtoxin-Injektionen führen nach wenigen Tagen zu einer dosisabhängigen Abnahme der Speichelproduktion, der Wirkbeginn liegt also bei wenigen Tagen. Effektivität, Wirkdauer und Nebenwirkungen sind überwiegend von der zu verabreichenden Dosis abhängig. Allgemein liegt die Wirkdauer zwischen vier und 16 Wochen, wobei die Wirkdauer abhängig vom verwandten Typ ist: so haben Typ E und F eine deutlich verkürzte Wirkdauer von ca. drei bis sechs Wochen. Botulinumtoxin schädigt die Speicheldrüsen nicht dauerhaft oder irreversibel. Die Speicheldrüsenfunktion wird unter Botulinumtoxin im Verlauf vollständig wiederhergestellt. Die Injektionen können ohne Organschädigung beliebig oft wiederholt werden

Der Wirkmechanismus von Botulinumtoxin an Speicheldrüsen ist bekannt und ausführlich vorbeschrieben. Alle Botulinumtoxin-Präparationen und Typen (A-G) induzieren 1-10 Tage, das heißt zeitlich variabel, nach Injektion eine dosisabhängige Denervierung der Drüsen und somit zeitlich befristete dosisabhängige Abnahme der Speichelsekretion. Typ B verfügt über einen vergleichsweise schnellen Wirkungsbeginn von etwa drei Tagen, während der Wirkungsbeginn bei Typ A ca. sechs Tage beträgt. Die Abnahme der Speichelsekretion betrifft sowohl seröse als auch muköse Drüsenanteile der jeweiligen Speicheldrüsen. Die Abnahme der Drüsenfunktion reduziert die Speichelproduktion und die Durchblutung der Drüsen. Über beide Mechanismen (einzeln oder kombiniert) kommt es zu einer verminderten Aufnahme der schädigenden Radionuklide durch verminderte Drüsenaktivität während der Radionuklid-PSMA-Therapie in den Drüsen und es wird die dauerhafte und irreversible Schädigung der Drüsen verhindert. Auch hier liegt ein Unterscheid zwischen Botulinumtoxin Typ B und Typ A vor: Typ B besitzt eine höhere Affinität zu autonomen Nervenendigungen als Typ A. Dies ist von besonderer Bedeutung, da die Speicheldrüsen (im Gegensatz zur Muskulatur) ausschließlich autonom innerviert werden. Daher wirkt Botulinumtoxin Typ B stärker und länger als Typ A an Speicheldrüsen. Die lokale Injektion mit Typ B im Vergleich zu Typ A führt zu einer größeren Ausbreitung im Körper mit einer höheren Affinität zu autonomen Strukturen. Dementsprechend kann Typ B bei Injektion entfernt liegender Drüsen zu autonomen Wirkungen an nicht injizierten Drüsen, wie beispielsweise den Augendrüsen führen. Daraus ergibt sich ein gewisser Schutzeffekt an den Augendrüsen durch Botulinumtoxin Typ B-Inj ektion in die Speicheldrüsen. Das heißt, eine Speicheldrüseninjektion mit Botulinumtoxin Typ B kann durch höhere Affinität zu autonomen Strukturen und eine größere regionale Ausbreitung an den ansonsten Botulinumtoxin-Injektionen nicht direkt zugänglichen Drüsen wie Unterzungenspeicheldrüse, kleine Speicheldrüsen sowie Augendrüsen eine gewisse Schutzfunktion ausüben. Dieser Wirkmechanismus gilt für alle mit BTX-behandelten Drüsen, auch wenn das Ausmaß der Denervierung mit Reduktion der Speichelproduktion bei den einzelnen Drüsen unterschiedlich stark ausgeprägt sein kann.

Ferner besitzen Speicheldrüsen Androgenrezeptoren. Der überwiegende Teil der Patienten mit fortgeschrittenem Prostatakarzinom erhalten eine antiandrogene Therapie. Antiandrogene erhöhen die PSMA-Aufnahme deutlich, was zu einer Potenzierung der Speicheldrüsenschädigung durch PSMA-Radioliganden führt. Daher ist die vorliegende Erfindung besonders geeignet für Patienten, die sich bereits einer antiandrogenen Therapie unterzogen haben oder sich einer Radioliganden-Therapie unterziehen, da sie besonders anfällig für eine Drüsenschädigung durch PSMA-Radioliganden sind.

Auch exprimieren alle Speicheldrüsen zahlreiche α-1-Adrenorezeptoren. Die Stimulation von α-1-Rezeptoren führt zu erheblicher Speicheldrüsenaktivierung und Speichelsekretion. Botulinumtoxin Typ A führt zu einer Herabregulation der α -1-Adrenorezeptoren und auch darüber hinaus zu einer reduzierten Speicheldrüsenaktivierung und zu einer reduzierten PSMA-Radioligandbindung an den mit Botulinumtoxin injizierten Speicheldrüsen.

Ferner führen Aquaporine insbesondere an der Unterkieferspeicheldrüse zu einer deutlich verstärkten PSMA-Radioligandenbindung. Aquaporine sind Proteine, die Kanäle in der Zellmembran bilden, um den Durchtritt von Wasser und einigen weiteren Molekülen wie Metalloiden zu erleichtern. Bei den Radionukliden handelt es sich überwiegend um Metalloide. Insbesondere die Unterkieferspeicheldrüse exprimiert in starkem Ausmaß Aquaporine (AQP) in der apikalen Membran der azinären Zellen der Speicheldrüsen und insbesondere der Unterkieferspeicheldrüse. Botulinumtoxin Typ A und Typ B reduzieren nach ca. ein bis zwei Wochen signifikant die Aquaporin mRNA (z.B. AQP5) in der Unterkieferspeicheldrüse und die AQP-Verteilung in der apikalen Membran. Zusätzlich induziert Botulinumtoxin Typ A und Typ B die Apoptose der Azini. Somit führen Botulinumtoxin-Injektionen in die Unterkieferspeicheldrüse zu einer signifikanten Abnahme der Aquaporin-vermittelten Radionuklidaufnahme in die Zelle und schützt somit die Speicheldrüsen, insbesondere die Unterkieferspeicheldrüse, vor permanenter Schädigung. Mit Abklingen der Botulinumtoxin-Wirkung bilden sich die genannten Aquaporin-Veränderungen vollständig zurück.

Insbesondere die häufig erforderliche Wiederholung der Radionuklid-PSMA-Therapie (in der Regel bis zu vier Behandlungen binnen sechs Monaten) führt zu einer kumulativen bleibenden massiven Schädigung der Speicheldrüsen. Die Botulinumtoxin-Wirkung klingt nach ca. sechs bis sechzehn Wochen folgenlos ab und die Speicheldrüsenfunktion setzt wieder ein.

Baum et al., 2018 ("Injection of Botulinum Toxin für Preventing Salivary Gland Toxicity after PSMA Radioligand Therapy: an Empirical Proofofa Promising Concept", Nuclear Medicine and Molecular Imaging, 2018) konnte durch die Applikation von Botulinum Toxin in die rechte Ohrspeicheldrüse eine Abnahme der Aufnahme des verwendeten Radioliganden in die rechte Ohrspeicheldrüse nachweisen. Der Effekt schien jedoch auf die rechte Ohrspeicheldrüse beschränkt.

Nicht nur Botulinumtoxin, auch Anticholinergika können die Speichel- und Augendrüsen schützen. Anticholinergika, auch Parasympatholytika genannt, unterdrücken die Wirkung von Acetylcholin, indem sie den Acetylcholinrezeptor kompetitiv hemmen. Damit werden die Nervenreize, die zur Sekretionssteigerung der Drüsen führen, blockiert. Somit ergänzen und potenzieren sich die Schutzwirkungen von Botulinumtoxin und Anticholinergika: Botulinumtoxin hemmt die Ausschüttung von Acetylcholin in den synaptischen Spalt (präsynaptisch) und die Anticholinergika blockieren die Acetylcholinrezeptoren (postsynaptisch). Erst die kombinierte prä- und postsynaptische Blockade durch beide Substanzen (Botulinumtoxin und Anticholinergika) ermöglicht die optimale Schutzwirkung an Speichel- und Augendrüsen während der Radioliganden-Therapie. Während einer Radioliganden-Therapie oder der Nutzung von Radioliganden zur Diagnostik erhöhen die parasympathischen Fasern die Speichelproduktion und es wird vor allem seröser Speichel in der Ohrspeicheldrüse produziert, daher wird diese Funktion durch die systemisch wirksamen Anticholinergika während der PSMA-Radionuklidtherapie besonders geschützt. Diese Wirkung wird in der vorliegenden Erfindung als Schutzwirkung der Drüsen zu Nutze gemacht. Anticholinergika sind dosisabhängig und systemisch effektiv und können daher Drüsen erreichen, die nicht direkt mit einer Botulinumtoxin-Injektion behandelbar sind, u.a. die Unterzungendrüse, die kleinen Speicheldrüsen sowie die Augendrüsen. Diese sind aufgrund ihrer anatomischen Lage und des Nebenwirkungsrisikos mit Botulinumtoxin nicht direkt behandelbar. Somit werden diese Drüsen, wie beispielsweise die Augendrüsen, durch Anticholinergika geschützt, so dass diese nicht durch einen Radioliganden dauerhaft geschädigt werden und somit stark die Lebensqualität einschränkende Austrocknung der Augen und chronische schwere Entzündungen von Augen und Lidrändern vermieden werden. Außerdem verstärken Anticholinergika die Botulinumtoxin-Wirkung an beispielsweise den Ohrspeicheldrüsen und der Unterkieferspeicheldrüse.

Insbesondere die Unterkieferspeicheldrüse profitiert von der Kombinationsbehandlung aus Botulinumtoxin und Anticholinergikum, da diese Drüse seromukös ist. Durch den doppelten Schutz wird gewährleistet, dass der Patient während des Kauvorgangs die Nahrung mechanisch in der Mundhöhle aufbereiten kann, der Nahrungsbrei weiterhin gut gleitet und die Verdauung nicht beeinträchtig wird. Ferner enthält Speichel antimikrobielle Bestandteile und sorgt so für die Remineralisation der Zähne, so dass Zahnschäden durch den doppelten Schutz vermieden werden.

Die Schutzwirkung auf die Drüsen kann durch eine Kombination von Botulinumtoxin und Anticholinergikum weiter verstärkt werden. Die Kombination mit einem Anticholinergikum führt für die Dauer der Wirksamkeit des jeweiligen Anticholinergikums zu einer Verstärkung der Botulinumtoxin-Wirkung an Ohrspeicheldrüse sowie Unterkieferspeicheldrüse und zu einer partiellen Wirkung an den übrigen Speicheldrüsen und Augendrüsen. Daher ist eine Kombination aus Botulinumtoxin und Anticholinergikum am effektivsten hinsichtlich der Speicheldrüsensekretion, gefolgt von Botulinumtoxin als Monotherapie, gefolgt von Anticholinergikum als Monotherapie.

Allerdings können nicht alle Patienten von einer Kombinationsbehandlung, bestehend aus Botulinumtoxin und Anticholinergikum, profitieren: Patienten mit einer kognitiven Störung, wobei alle Schweregerade einer kognitiven Störung beinhaltet sind (von leichter, über mittel schwerer bis schwerer kognitiver Störung) dürfen kein Anticholinergikum einnehmen, da kognitionsverändernde Effekte auftreten können. In anderen Worten, es besteht eine Kontraindikation. Ebenso dürfen Patienten mit Herzrhythmusstörungen nicht mit Anticholinergika behandelt werden, weil Anticholinergika die Herzfrequenz erhöhen und es auch zu Überleitungsstörungen, Tachyarrhythmien, Herzinsuffizienz und Angina-Pectoris-Anfällen kommen kann. Auch hier besteht eine Kontraindikation. Daher werden diese Patientengruppen, die einer Radioliganden-Diagnostik oder -Therapie ausgesetzt werden, mit Botulinumtoxin ohne Anticholinergikum behandelt. Hier entfaltet das Botulinumtoxin A seine Wirkung sowohl direkt in der injizierten Drüse, als auch per passive Diffusion in benachbarten Drüsen. Wird beispielsweise beidseits die Unterkieferspeicheldrüse mit Botulinumtoxin Typ A injiziert, so wird auch die Unterzungenspeicheldrüse per Diffusion geschützt. Botulinumtoxin B entfaltet seine Wirkung ebenso direkt in der injizierten Drüse, als auch per passive Diffusion in weiteren Drüsen, wobei der Diffusionsradius im Vergleich zu Botulinumtoxin Typ A größer ist. Zusätzlich verfügt Botulinumtoxin Typ B über eine höhere Affinität zu autonomen Strukturen, was dazu führt, dass Botulinumtoxin Typ B auch an entfernt liegende Drüsen, die nicht mit Botulinumtoxin Typ B injiziert wurden, wie beispielsweise den Augendrüsen, der Unterzungenspeicheldrüse und den kleinen Speicheldrüsen, wirkt.

Zusammenfassend wird dringend ein Verfahren zum Schutz insbesondere der lebenswichtigen Augen- und Speicheldrüsen, welche im Rahmen der Diagnostik oder Therapie nicht die Zielstruktur der Radioliganden-Diagnostik oder Radioliganden-Therapie darstellen, benötigt.

Das vorliegende Verfahren der Erfindung bietet eine präventive Lösung an, um einer irreversiblen Drüsenschädigung durch diagnostische oder therapeutische Radioliganden vorzubeugen.

### Zusammenfassung der Erfindung

Die vorliegende Erfindung bezieht sich auf Botulinumtoxin in Kombination mit einem Anticholinergikum ausgewählt aus Tropicamid, Atropin, Scopolamin, Glycopyrrolat, Amitriptylin, Clonidin, Ipratropium-Bromid und Trihexyphenidyl zur Verwendung in einem Verfahren zur Prävention von Strahlenschäden der Drüsen genutzt, wobei die Strahlenschäden von Radioliganden hervorgerufen werden. Ferner umfasst die vorliegende Erfindung auch Botulinumtoxin Typ A oder Typ B zur Verwendung in einem Verfahren zur Prävention von Strahlenschäden der Drüsen genutzt, wobei die Strahlenschäden von Radioliganden hervorgerufen werden und wobei das Botulinumtoxin Typ A oder Typ B in die jeweilige Ohrspeichel- und kontralaterale Unterkieferspeicheldrüse zu verabreichen ist.

In manchen Ausführungsformen wird Botulinumtoxin in Kombination mit einem Anticholinergikum zur Verwendung in einem Verfahren genutzt, wobei die Strahlenschäden von PSMA-Radioliganden hervorgerufen werden

In manchen Ausführungsformen wird Botulinumtoxin in Kombination mit einem Anticholinergikum zur Verwendung in einem Verfahren genutzt, wobei die Strahlenschäden von ²²⁵Ac-PSMA-617, ⁶⁸Ga-PSMA, ¹⁸F-PSMA oder ¹⁷⁷Lu-PSMA hervorgerufen werden.

In manchen Ausführungsformen wird das Botulinumtoxin aus der Gruppe der Botulinumtoxin-Typen A, B, C, D, E, F und G ausgewählt.

In manchen Ausführungsformen ist das Botulinumtoxin ein Botulinumtoxin Typ A.

In manchen Ausführungsformen umfasst das Botulinumtoxin zwischen 1 und 10.000 Einheiten.

In manchen Ausführungsformen umfasst das Botulinumtoxin zwischen 1 Einheit und 1.500 Einheiten von Typ A.

In manchen Ausführungsformen ist das Botulinumtoxin ein Botulinumtoxin Typ B.

In manchen Ausführungsformen umfasst das Botulinumtoxin zwischen 100 und 10.000 Einheiten von Typ B.

In manchen Ausführungsformen ist das Botulinumtoxin ein Botulinumtoxin Typ E oder Typ F.

In manchen Ausführungsformen umfasst das Botulinumtoxin 1 und 10.000 Einheiten von Typ E oder Typ F.

In manchen Ausführungsformen wird das Anticholinergikum transdermal, oral oder intravenös zu verabreichen ist.

In manchen Ausführungsformen ist das Anticholinergikum Scopolamin, wobei das Scopolamin transdermal zu verabreichen ist.

In manchen Ausführungsformen ist das Botulinumtoxin Botulinumtoxin Typ B zur Prävention von Strahlenschäden der Ohrspeichel-, Unterkieferspeichel-, Unterzungenspeichel-, kleinen Speichel- und Augen-Drüsen, wobei die Strahlenschäden von Radioliganden hervorgerufen werden.

In manchen Ausführungsformen ist das Botulinumtoxin Botulinumtoxin Typ A zur Herabsetzung der Aquaporin-vermittelten Radionuklidaufnahme in den Zellen den Unterkieferspeicheldrüsen und Speicheldrüsen.

In manchen Ausführungsformen ist das Botulinumtoxin Botulinumtoxin Typ A, wobei das Botulinumtoxin Typ A in einem Dosisverhältnis von 2/3 in die Ohrspeicheldrüsen und zu 1/3 in die Unterkieferspeicheldrüsen zu verabreichen ist.

In manchen Ausführungsformen ist das Botulinumtoxin Botulinumtoxin Typ B, wobei das Botulinumtoxin Typ B in einem Dosisverhältnis von 2/3 in die Ohrspeicheldrüsen und zu 1/3 in die Unterkieferspeicheldrüsen zu verabreichen ist.

In manchen Ausführungsformen umfasst die Verwendung die Verabreichung von Botulinumtoxin ein Tag bis acht Wochen vor einem bildgebenden Verfahren oder vor einer radiologischen Behandlung, welche ein oder mehrere Radionuklide verwenden.

In manchen Ausführungsformen umfasst die Verwendung die Verabreichung des Anticholinergikums drei Tage, zwei Tage, ein Tag und am gleichen Tag vor einem bildgebenden Verfahren zur Diagnostik oder vor einer radiologischen Behandlung, welche ein oder mehrere Radionuklide verwenden, sowie etwa 7 bis 30 Tage nach dem bildgebenden Verfahren zur Diagnostik oder nach einer radiologischen Behandlung.

In manchen Ausführungsformen umfasst die Verwendung die Verabreichung des Botulinumtoxins einen Tag bis acht Wochen und des Anticholinergikums drei Tage, zwei Tage, ein Tag und am gleichen Tag vor einem bildgebenden Verfahren zur Diagnostik oder vor einer radiologischen Behandlung, welches ein oder mehrere Radionuklide verwendet, sowie etwa 7 bis 30 Tage nach dem bildgebenden Verfahren zur Diagnostik oder nach einer radiologischen Behandlung.

In manchen Ausführungsformen sind die Drüsen exokrine und/oder endokrine Drüsen.

In manchen Ausführungsformen ist die Drüse eine seromuköse Drüse.

In manchen Ausführungsformen wirkt das Botulinumtoxin Typ A durch die Herabregulierung der α-1-Adrenorezeptoren in den Drüsen.

In manchen Ausführungsformen wird die Verwendung bevorzugt in einem Patientenkollektiv durchgeführt, das sich bereits einer Anti-Androgentherapie unterzogen hat oder während der Radionuklidtherapie einer Anti-Androgentherapie unterzieht.

### Vorteile der Erfindung

Durch die Verwendung von Botulinumtoxin und einem Anticholinergikum im Verfahren der vorliegenden Erfindung werden Speicheldrüsen und Augendrüsen geschützt. Das heißt, permanente und irreversible Nebenwirkungen wie Mundtrockenheit, Ulcera im gesamten Mundbereich, Schluckstörungen, Aspirationsgefahr, Zahnschäden, werden verhindert und somit auch eine weitere Behandlung dieser Nebenwirkungen. Daher wird das Gesundheitssystem entlastet. Im schlimmsten Fall können starke Nebenwirkungen in eine Dosisanpassung oder gar einen Abbruch einer Radioligand-Therapie resultieren. Auch diese Szenarien werden mit der vorliegenden Erfindung verhindert.

In manchen Ausführungsformen umfasst das Verfahren die Verwendung von Botulinumtoxin und Anticholinergikum. Dies erhöht die Schutzwirkung auf die Drüsen weiter. Die Kombination mit einem Anticholinergikum führt für die Dauer der Wirksamkeit des jeweiligen Anticholinergikums zu einer Verstärkung der Botulinumtoxin-Wirkung an Ohrspeicheldrüsen und Unterkieferspeicheldrüse und zusätzlich zu einer Schutzwirkung an den übrigen Speicheldrüsen und Augendrüsen. Das heißt, permanente und irreversible Nebenwirkungen wie trockenes Auge, wiederholte Augenentzündungen, Sehstörungen, Verlust an Sehschärfe werden zusätzlich zu Mundtrockenheit, Ulcera im gesamten Mundbereich, Schluckstörungen, Aspirationsgefahr und Zahnschäden, verhindert und somit auch eine weitere Behandlung dieser Nebenwirkungen.

### Abbildungen

Die folgenden Abbildungen stellen exemplarisch bevorzugte Therapieschemata dar. Das heißt, dass auch andere Therapieschema möglich und von der vorliegenden Erfindung umfasst sind. Dies bedeutet, dass auch Monotherapien mit Botulinumtoxin Typ A oder Typ B exemplarische Therapieschemata darstellen.

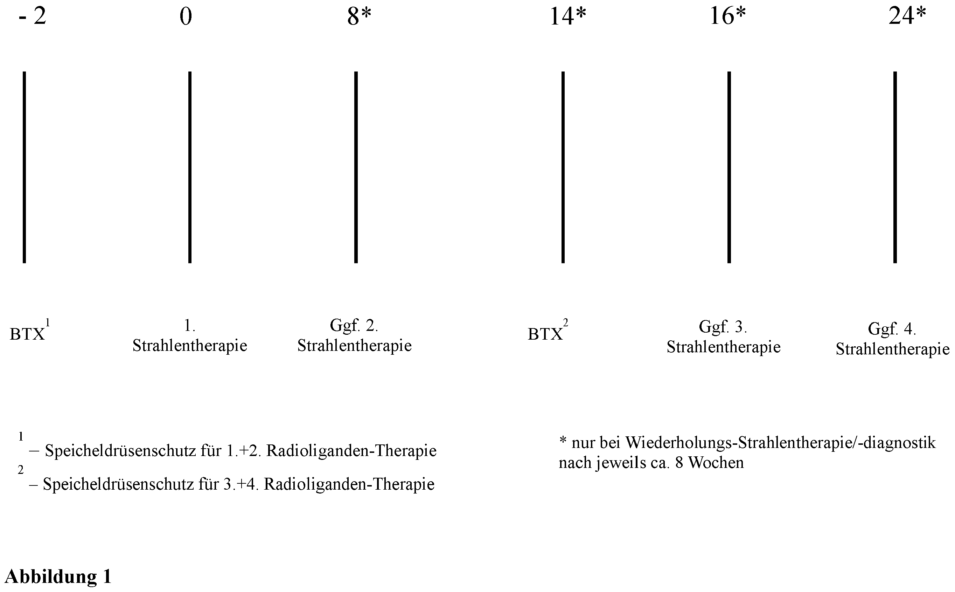

**Abbildung 1****:** Therapieschema zur Prävention von Strahlenschäden durch die Verwendung von Botulinumtoxin Typ A oder Typ B bei der therapeutischen Verwendung von Radioliganden (alle acht Wochen, in diesem Beispiel vier Verabreichungen des Radioliganden im Rahmen des therapeutischen Verfahrens dargestellt, Verabreichungen können mehr als vier Mal stattfinden)

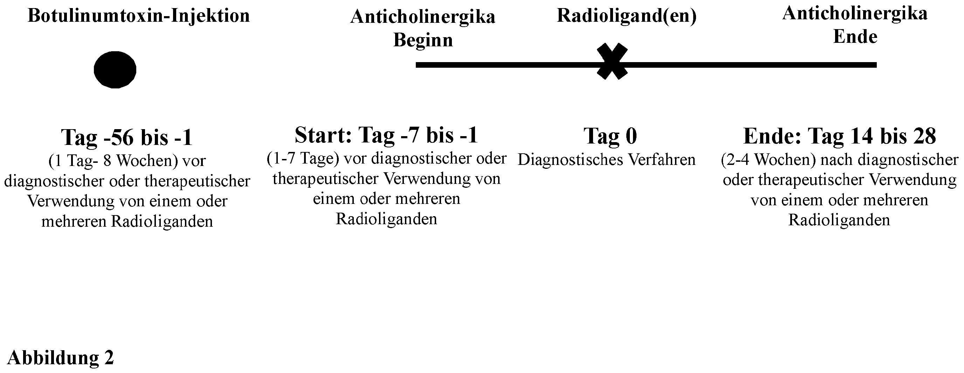

**Abbildung 2****:** Therapieschema zur Prävention von Strahlenschäden durch die Verwendung von Botulinumtoxin Typ A oder B und einem Anticholinergikum bei der diagnostischen und ersten therapeutischen Verwendung von Radioliganden

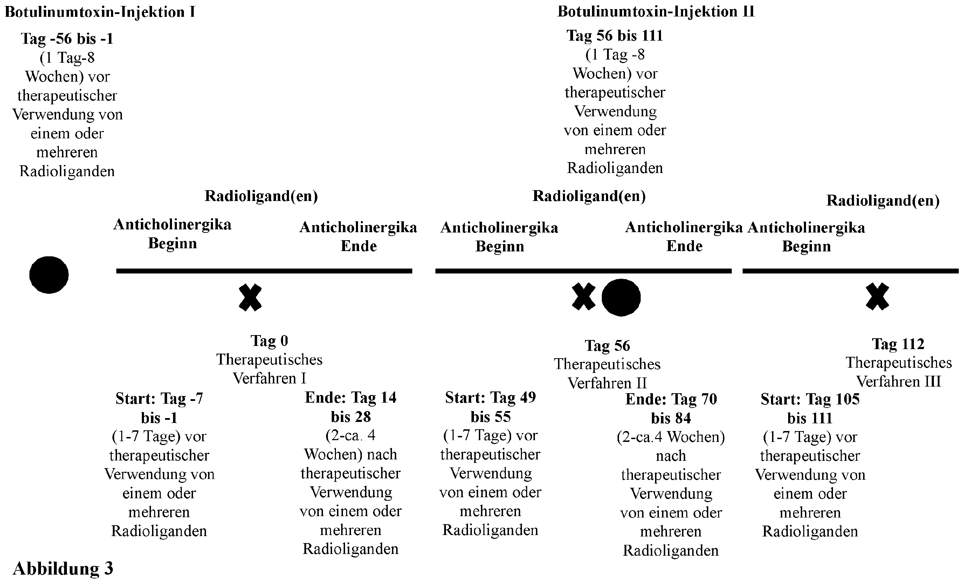

**Abbildung 3****:** Therapieschema zur Prävention von Strahlenschäden durch die Verwendung von Botulinumtoxin Typ A oder Typ B und einem Anticholinergikum bei der therapeutischen Verwendung von Radioliganden (alle 56 Tage, in diesem Beispiel drei von vier Verabreichungen des Radioliganden im Rahmen des therapeutischen Verfahrens dargestellt).

### Ausführliche Beschreibung

### Definitionen:

**"Botulinumtoxin"** ist ein Sammelbegriff für die Familie von Toxinen, die durch das Bakterium *Clostridium botulinum* hergestellt werden, darstellt. Aktuell sind sieben unterschiedliche Botulinumtoxine (A, B, C, D, E, F und G) bekannt, die sich in ihren Eigenschaften voneinander unterscheiden. Botulinumtoxin bedeutet in der vorliegenden Anmeldung, dass alle unterschiedlichen Typen umfasst sind. In der klinischen Verwendung befinden sich aktuell drei Botulinumtoxinpräparate vom Typ A und ein Botulinumtoxinpräparat vom Typ B.

**"Anticholinergika"** ist ein Sammelbegriff für Wirkstoffe, welche den Effekt von dem Neurotransmitter Acetylcholin an den Effektor-Zellen antagonisieren, die initiale Ausschüttung von Acetylcholin bleibt unberührt.

**"Prävention"** auch Vorbeugung genannt, bezeichnet Maßnahmen zur Abwendung von unerwünschten Ereignissen oder Zuständen, die mit einer gewissen Wahrscheinlichkeit eintreffen könnten, wenn nichts getan würde. Im Zusammenhang mit dieser Anmeldung bedeutet Prävention, dass Schäden beziehungsweise Nebenwirkungen an humanen Drüsen und anderen Körperstellen vermieden werden, die durch die Diagnose oder Therapie mit Radioliganden entstehen könnten. Diese Schäden umfassen beispielsweise Mundtrockenheit, Ulcera im gesamten Mundbereich, Schluckstörungen, Aspirationsgefahr, Zahnschäden, Augentrockenheit, Sehstörungen sowie (chronische) Augenentzündungen und Verlust an Sehschärfe.

**"Strahlenschäden"** sind Schäden, die ionisierende Strahlen an Organismen verursachen. Die Wirkung von ionisierender Strahlung im Organismus kann aus einer Reihe von physikalischen, chemischen, biochemischen und biologischen Prozessen bestehen. Im Rahmen der vorliegenden Erfindung sind Schäden beziehungsweise Nebenwirkungen, hervorgerufen durch die Nutzung von Radioliganden für Diagnostik und/oder Therapie gemeint. Diese Schäden beziehungsweise Nebenwirkungen umfassen beispielsweise Mundtrockenheit, Ulcera im gesamten Mundbereich, Schluckstörungen, Aspirationsgefahr, Zahnschäden, Augentrockenheit, Sehstörungen sowie (chronische) Augenentzündungen, und Verlust an Sehschärfe.

**"Radionuklid"** ist ein Nuklid, wenn es instabil und damit radioaktiv ist. Radionuklide der vorliegenden Erfindung sind Radionuklide, die zu medizinischen Zwecken, vor allem in der Diagnostik wie bei bildgebenden Verfahren oder zur Therapie eingesetzt werden.

**"Radioligand"** ist eine mit einem Radionuklid markierte Substanz, die als ein Ligand an ein Zielprotein, beispielsweise ein Rezeptor, binden kann. Solche Radioliganden werden beispielsweise sowohl in der Diagnostik als auch in der Therapie von zum Beispiel onkologischen Erkrankungen eingesetzt.

**"Drüsen"** sind Organe und im weiteren Sinne auch einzelne Zellen, die in der Lage sind, spezifische Substanzen zu synthetisieren und sezernieren. Drüsen können über seröse und muköse Anteile gleichzeitig verfügen, welche einen Einfluss auf die produzierte Substanz haben. Nicht-limitierende Beispiele für solche Substanzen stellen Speichel oder Tränen dar. Eine weitere Klassifizierung von Drüsen stellt die Kategorisierung von Drüsen in exokrine und endokrine Drüsen dar.

**"Dosisverhältnis"** Als Dosis wird in der Regel die Menge eines pharmazeutischen Wirkstoffs oder eines Arzneimittels bezeichnet, die für die Verabreichung vorgesehen ist, hier die Menge des Botulinumtoxins. Dosisverhältnisse können als Fraktionen (z.B. 1/3) oder als Verhältnis wie 1: 1, 1:2 etc. angegeben werden.

**"Kognitive Störungen"** sind Gedächtnisstörungen jeglicher Ausprägung. Somit sind leichte, mittelschwere und schwere kognitive Störungen umfasst. Eine leichte kognitive Störung oder eine leichte kognitive Beeinträchtigung (auch als mild cognitive impairment", MCI bezeichnet) ist eine Beeinträchtigung der Denkleistung, die über das nach Alter und Bildung des Betroffenen Normale hinausgeht, jedoch im Alltag keine wesentliche Behinderung darstellt.

**"Reduzierte Lebenserwartung"** Eine reduzierte Lebenserwartung beträgt zwischen sechs und achtzehn Monaten, bevorzugterweise sechs Monate ab Diagnosestellung.

**"Hochdosistherapie"** Unter einen Hochdosistherapie versteht man die Applikation von insgesamt zwischen 100 und 300 Einheiten Botulinumtoxin Typ A, besonders bevorzugt von insgesamt 150 Einheiten Botulinumtoxin Typ A. Dabei werden zwischen 75 und 150 Einheiten, besonders bevorzugt 100 Einheiten in eine Ohrspeicheldrüse appliziert und zwischen 25 und 75 Einheiten, besonders bevorzugt 50 Einheiten in eine Unterkieferspeicheldrüse appliziert.

Besonders bevorzugt wird das hochdosierte Botulinumtoxin Typ A asymmetrisch (kontralateral) appliziert.

**"Asymmetrische (kontralaterale) Applikation"** Bei einer asymmetrischen (kontralateralen) Applikation wird das Botulinumtoxin in eine Drüse und in jene Drüse, die auf der entgegengesetzten Körperseite liegt, injiziert. Beispielsweise wird das Botulinumtoxin in die rechte Ohrspeicheldrüse und in die (kontralaterale) linke Unterkieferspeicheldrüsen (50 Einheiten) appliziert. Das heißt, es kann beispielsweise die rechte Ohrspeicheldrüse und die linke Unterkieferspeicheldrüse zur Injektion ausgewählt werden oder *vice versa.*

**"Demenz"** Unter einer Demenz versteht man eine Sammlung von Symptomen unterschiedlicher Erkrankungen, deren Hauptmerkmal eine Verschlechterung von mehreren kognitiven Fähigkeiten im Vergleich zum früheren Zustand ist. Eine Demenz kann durch diverse degenerative und nichtdegenerative Erkrankungen des Gehirns entstehen.

**"Komorbid" oder "multimorbid"** bedeutet, dass ein Patient an einem weiteren Krankheitsbild oder Syndrom zusätzlich zu einer Grunderkrankung leidet, beziehungsweise gleichzeitig an mehreren, verschiedenen Erkrankungen leidet.

### Ausführungsformen der Erfindung

Das Verfahren der vorliegenden Erfindung ist für eine Vielzahl von Verwendungszwecken geeignet. Es eignen sich nicht nur zur Diagnostik von Erkrankungen, für welche Radionuklide bzw. Radioliganden verwandt werden, sondern auch zur Behandlung von Erkrankungen, für welche Radionuklide bzw. Radioliganden verwandt werden. Beispielhafte Radionuklide umfassen ³²P, ⁶⁰Co, ⁹⁰Sr, ⁹⁰Y, ¹⁰³Pd, ¹²⁵I, ¹³¹I, ¹³⁷Cs, ¹⁸⁸Re, ¹⁹²Ir, ¹⁹⁸Au, ²²⁶Ra. Erkrankungen umfassen onkologische Erkrankungen wie Bronchialkarzinome, wie das kleinzellige oder das großzellige Bronchialkarzinom, Dickdarmkrebs, Brustkrebs, Prostatakrebs, Leberkrebs, Pankreaskrebs, Blasenkrebs, Hautkrebs, Eierstockkrebs, Krebsarten des Urogenitaltraktes, Nebennierenrindenkrebs (Phäochromozytom), Krebserkrankungen des Gehirns, Magenkrebs, Nierenkrebs, Uteruskrebs, Knochenkrebs, Ösophaguskrebs, oropharyngale Krebserkrankungen, Hodenkrebs, Schilddrüsenkrebs, adenokortikale Krebserkrankungen, Gallenblasenkrebs, Dünndarmkrebs, Analkrebs, Bauchspeicheldrüsenkrebs, Gallengangkrebs, Gebärmutterhalskrebs, Gebärmutterkörperkrebs, Harnröhrenkrebs, Kehlkopfkrebs, Knochenkrebs, Wilms-Tumor, Plasmocytom, Hodgkin Lymphom, Non-Hodgkin-Lymphom oder Retinoblastom. Andere Erkrankungen sind ebenfalls umfasst, welche die Diagnostik oder Therapie mittels Radioliganden erfordern.

Die vorliegende Erfindung bezieht sich auf Botulinumtoxin in Kombination mit einem Anticholinergikum ausgewählt aus Tropicamid, Atropin, Scopolamin, Glycopyrrolat, Amitriptylin, Clonidin, Ipratropium-Bromid und Trihexyphenidyl zur Verwendung in einem Verfahren zur Prävention von Strahlenschäden der Drüsen genutzt, wobei die Strahlenschäden von Radioliganden hervorgerufen werden. Radioliganden im Sinne der Erfindung umfassen sämtliche Radionuklide, die an einen Liganden gekoppelt sind.

Die vorliegende Erfindung bezieht sich ferner auf Botulinumtoxin Typ B zur Verwendung in einem Verfahren zur Prävention von Strahlenschäden der Ohrspeichel- und Unterkieferspeicheldrüsen, wobei die Strahlenschäden von Radioliganden hervorgerufen werden und wobei das Botulinumtoxin Typ B in die jeweilige Ohrspeicheldrüse und die kontralaterale Unterkieferspeicheldrüse zu verabreichen ist.

Daneben bezieht sich die Erfindung auf Botulinumtoxin Typ A zur Verwendung in einem Verfahren zur Prävention von Strahlenschäden der Drüsen, wobei die Strahlenschäden von Radioliganden hervorgerufen werden und wobei das Botulinumtoxin Typ A in die jeweilige Ohrspeicheldrüse und in die kontralaterale Unterkieferspeicheldrüse zu verabreichen ist.

In manchen Ausführungsformen wird Botulinumtoxin in Kombination mit einem Anticholinergikum zur Verwendung in einem Verfahren genutzt, wobei die Strahlenschäden von PSMA-Radioliganden hervorgerufen werden. Ein besonders bevorzugter PSMA-Radioligand ist PSMA-617.

In manchen Ausführungsformen wird Botulinumtoxin in Kombination mit einem Anticholinergikum zur Verwendung in einem Verfahren genutzt, wobei die Strahlenschäden von ²²⁵Ac-PSMA-617, ⁶⁸Ga-PSMA, ¹⁸F-PSMA oder ¹⁷⁷Lu-PSMA hervorgerufen werden. In manchen Ausführungsformen ist das PSMA an andere diagnostisch oder therapeutisch geeignete Radionuklide gekoppelt wie beispielsweise ¹¹¹In.

In manchen Ausführungsformen wird das Botulinumtoxin aus der Gruppe der Botulinumtoxin-Typen A, B, C, D, E, F und G ausgewählt. In manchen Ausführungsformen ist ein Botulinumtoxin Typ A bevorzugt. In manchen Ausführungsformen ist ein Botulinumtoxin Typ B bevorzugt. In weiteren Ausführungsformen ist eine Kombination der verschiedenen Botulinumtoxin-Typen bevorzugt, wie Typ A und Typ B, Typ A und E, Typ A und F, Typ B und E, Typ B und F. Ferner umfasst sind modifizierte, rekombinante sowie synthetische Botulinumtoxine. Modifizierte Botulinumtoxine umfassen Aminosäuresubstitutionen, Deletionen oder Insertionen, die in Codons eines Polynukleotid vorgenommen werden, das wiederum für das modifizierte Polypeptid kodiert. Ferner umfasst sind chemische Modifikationen, wie beispielsweise die PEGylierung des Botulinumtoxins oder Phosphorylierungen der Aminosäuren. Rekombinante Botulinumtoxine sind rekombinant hergestellte Botulinumtoxine, Methoden der rekombinanten DNA-Technologie zur Herstellung solcher rekombinanten Botulinumtoxine sind hinreichend bekannt. Synthetische Botulinumtoxine sind Polypeptide mit definierter Sequenz. Synthetische Botulinumtoxine können sowohl die exakte Kopie des natürlich vorkommenden Botulinumtoxins darstellen, als auch in diversen Modifikationen erzeugt werden können. So können synthetische Botulinumtoxine beispielsweise unnatürlichen Aminosäuren umfassen oder auch die Modifikation der Peptid-Hauptkette.

In manchen Ausführungsformen umfasst das Botulinumtoxin zwischen 1 und 10.000 Einheiten, bevorzugt zwischen 10 und 5000 Einheiten, und am bevorzugten zwischen 20 und 4.000 Einheiten.

In manchen Ausführungsformen umfasst das Botulinumtoxin zwischen 1 Einheit und 1.500, bevorzugt zwischen 10 und 1.000 Einheiten und am bevorzugten zwischen 20 und 900 Einheiten von Typ A.

In manchen Ausführungsformen umfasst das Botulinumtoxin zwischen 100 und 10.000 Einheiten, bevorzugt zwischen 250 und 5.000 Einheiten und am bevorzugten zwischen 500 und 4.000 Einheiten von Typ B.

In manchen Ausführungsformen ist das Botulinumtoxin ein Botulinumtoxin Typ E oder Typ F.

In manchen Ausführungsformen umfasst das Botulinumtoxin zwischen 1 Einheit und 10.000 Einheiten von Typ E oder Typ F.

Das Anticholinergikum ist ausgewählt aus der Gruppe bestehend aus Tropicamid, Atropin, Scopolamin, Glycopyrrolat, Amitriptylin, Clonidin, Ipratropium-Bromid und Trihexyphenidyl. Besonders bevorzugt ist hierbei Scopolamin. In manchen Ausführungsformen wird das Anticholinergikum transdermal, oral oder intravenös verabreicht. Bevorzugterweise wird das Anticholinergikum transdermal verabreicht. Am Bevorzugtesten wird Scopolamin transdermal verabreicht.

In manchen Ausführungsformen ist das Botulinumtoxin Botulinumtoxin Typ B zur Prävention von Strahlenschäden der Ohrspeichel-, Unterkieferspeichel-, Unterzungenspeichel-, kleinen Speichel- und Augen-Drüsen, wobei die Strahlenschäden von Radioliganden hervorgerufen werden. In manchen Ausführungsformen wird das Botulinumtoxin Typ B in einem Verfahren verwandt, in welchem Patienten, die sich einer Radionuklid-Diagnostik oder -Behandlung unterziehen, unter einer kognitiven Störung leiden. In manchen Ausführungsformen wird das Botulinumtoxin Typ B in einem Verfahren verwandt, in welchem Patienten, die sich einer Radionuklid-Diagnostik oder -Behandlung unterziehen, unter einer Herzrhythmusstörung leiden. In einer bevorzugten Ausführungsform wird Botulinumtoxin Typ B in einem Verfahren verwandt, in welchem Patienten, die sich einer Radionuklid-Diagnostik oder -Behandlung unterziehen, unter einer kognitiven Störung und einer Herzrhythmusstörung leiden.

In manchen Ausführungsformen ist das Botulinumtoxin Botulinumtoxin Typ A zur Herabsetzung der Aquaporin-vermittelten Radionuklidaufnahme in den Zellen der Unterkieferspeicheldrüse und Speicheldrüse. In manchen Ausführungsformen wird das Botulinumtoxin Typ A in einem Verfahren verwandt, in welchem Patienten, die sich einer Radionuklid-Diagnostik oder -Behandlung unterziehen, unter einer kognitiven Störung leiden. In manchen Ausführungsformen wird das Botulinumtoxin Typ A in einem Verfahren verwandt, in welchem Patienten, die sich einer Radionuklid-Diagnostik oder -Behandlung unterziehen, unter einer Herzrhythmusstörung leiden. In einer weiteren Ausführungsform wird Botulinumtoxin Typ A in einem Verfahren verwandt, in welchem Patienten, die sich einer Radionuklid-Diagnostik oder -Behandlung unterziehen, unter einer kognitiven Störung und einer Herzrhythmusstörung leiden.

In einer Ausführungsform ist das Botulinumtoxin Botulinumtoxin Typ A, wobei das Botulinumtoxin Typ A in einem Dosisverhältnis von 1:1, 2:1, 3:1, 4:1, 5:1 in die Ohrspeicheldrüsen und in die Unterkieferspeicheldrüsen zu verabreichen ist. In einer besonders bevorzugten Ausführungsform ist das Botulinumtoxin Botulinumtoxin Typ A, wobei das Botulinumtoxin Typ A in einem Dosisverhältnis von 2/3 in die Ohrspeicheldrüsen und zu 1/3 in die Unterkieferspeicheldrüsen zu verabreichen ist, in anderen Worten, in einem Verhältnis von 2:1. In einer besonders bevorzugten Ausführungsform wird das Botulinumtoxin Typ A hochdosiert, oder in anderen Worten als "Hochdosistherapie" verabreicht. Dabei werden insgesamt zwischen 100 und 300 Einheiten Botulinumtoxin Typ A appliziert, besonders bevorzugt 150 Einheiten Botulinumtoxin Typ A appliziert. Es werden zwischen 75 und 150 Einheiten, besonders bevorzugt 100 Einheiten in eine Ohrspeicheldrüse appliziert und zwischen 25 und 75 Einheiten, besonders bevorzugt 50 Einheiten in eine Unterkieferspeicheldrüse appliziert. Besonders bevorzugt wird das hochdosierte Botulinumtoxin Typ A kontralateral (asymmetrisch) appliziert. Das Botulinumtoxin Typ A wurde asymmetrisch (100 Einheiten) in die rechte Ohrspeicheldrüse und in die kontralaterale, linke Unterkieferspeicheldrüsen (50 Einheiten) appliziert. Das heißt, es kann beispielsweise die rechte Ohrspeicheldrüse und die linke Unterkieferspeicheldrüse zur Injektion ausgewählt werden oder *vice versa.*

In einer Ausführungsform ist das Botulinumtoxin Botulinumtoxin Typ B, wobei das Botulinumtoxin Typ B in einem Dosisverhältnis von 1:1, 2:1, 3:1, 4:1, 5:1 in die Ohrspeicheldrüsen und in die Unterkieferspeicheldrüsen zu verabreichen ist. In einer besonders bevorzugten Ausführungsform ist das Botulinumtoxin Botulinumtoxin Typ B, wobei das Botulinumtoxin Typ B in einem Dosisverhältnis von 2/3 in die Ohrspeicheldrüsen und zu 1/3 in die Unterkieferspeicheldrüsen zu verabreichen ist, in anderen Worten, in einem Verhältnis von 2:1.

In manchen Ausführungsformen umfasst die Verwendung die Verabreichung von Botulinumtoxin 1 Tag bis sechzehn Wochen vor einem bildgebenden Verfahren oder vor einer radiologischen Behandlung, welche ein oder mehrere Radionuklide verwenden. In bevorzugten Ausführungsformen umfasst die Verwendung die Verabreichung von Botulinumtoxin 1 Tag bis acht Wochen vor einem bildgebenden Verfahren oder vor einer radiologischen Behandlung, welche ein oder mehrere Radionuklide verwenden. Hierbei ist der Zeitpunkt der Verabreichung abhängig vom verwandten Botulinumtoxin Typen: Typ E und Typ F werden 1 Tag bis acht Wochen vor einem bildgebenden Verfahren oder vor einer radiologischen Behandlung verbreicht, bevorzugter 1 Tag bis 4 Wochen, noch bevorzugter 1 Tag bis 2 Wochen, am bevorzugtesten 1 Tag bis 7 Tage vor einem bildgebenden Verfahren oder vor einer radiologischen Behandlung, welche ein oder mehrere Radionuklide verwenden. Typ A und B werden drei Tage bis zwölf Wochen vor einem bildgebenden Verfahren oder vor einer radiologischen Behandlung verbreicht, bevorzugterweise drei Tage bis vier Wochen vor einem bildgebenden Verfahren oder vor einer radiologischen Behandlung, welche ein oder mehrere Radionuklide verwenden, ebenso bevorzugt zwischen zwei und vier Wochen, am bevorzugtesten zwei Wochen vor einem bildgebenden Verfahren oder vor einer radiologischen Behandlung.

In manchen Ausführungsformen umfasst die Verwendung die Verabreichung des Anticholinergikums sieben Tage, sechs Tage, fünf Tage, vier Tage, drei Tage, zwei Tage, einen Tag und am gleichen Tag vor einem bildgebenden Verfahren zur Diagnostik oder vor einer radiologischen Behandlung, welche ein oder mehrere Radionuklide verwenden, sowie etwa bis zu 14 bis 28 Tagen nach dem bildgebenden Verfahren zur Diagnostik oder nach einer radiologischen Behandlung. In manchen Ausführungsformen umfasst die Verwendung, die Verabreichung des Anticholinergikums sieben Tage, sechs Tage, fünf Tage, vier Tage, drei Tage, zwei Tage, einen Tag und am gleichen Tag vor einem bildgebenden Verfahren zur Diagnostik oder vor einer radiologischen Behandlung, welche ein oder mehrere Radionuklide verwenden, sowie etwa bis zu 14 bis 28 Tage nach dem bildgebenden Verfahren zur Diagnostik oder nach einer radiologischen Behandlung. Das Anticholinergikum kann wie zuvor erwähnt, verschiedene Darreichungsformen aufweisen. Wenn das Anticholinergikum in der vorliegenden Erfindung transdermal appliziert wird, so kann die Verabreichung täglich, jeden zweiten Tag, jeden dritten Tag oder auch jeden vierten Tag erfolgen.

In manchen Ausführungsformen umfasst die Verwendung die Verabreichung des Botulinumtoxins vier Wochen und des Anticholinergikums sieben Tage, sechs Tage, fünf Tage, vier Tage, drei Tage, zwei Tage, einen Tag und am gleichen Tag vor einem bildgebenden Verfahren zur Diagnostik oder vor einer radiologischen Behandlung, welches ein oder mehrere Radionuklide verwendet, sowie bis zu 28 Tage nach dem bildgebenden Verfahren zur Diagnostik oder nach einer radiologischen Behandlung. In bevorzugten Ausführungsformen umfasst die Verwendung die Verabreichung des Botulinumtoxins zwei Wochen und des Anticholinergikums sieben Tage, sechs Tage, fünf Tage, vier Tage, drei Tage, zwei Tage, einen Tag und am gleichen Tag vor einem bildgebenden Verfahren zur Diagnostik oder vor einer radiologischen Behandlung, welches ein oder mehrere Radionuklide verwendet, sowie bis zu 28 Tage nach dem bildgebenden Verfahren zur Diagnostik oder nach einer radiologischen Behandlung. Das Anticholinergikum kann wie zuvor erwähnt, verschiedene Darreichungsformen aufweisen. Wenn das Anticholinergikum in der vorliegenden Erfindung transdermal appliziert wird, so kann die Verabreichung täglich, jeden zweiten Tag, jeden dritten Tag oder auch jeden vierten Tag erfolgen. In einer bevorzugten Ausführungsform weisen jene Patienten, die sowohl mit Botulinumtoxin und einem Anticholinergikum behandelt werden, ein mittleres Lebensalter zwischen 50 und 75 Jahren auf.

In manchen Ausführungsformen sind die Drüsen exokrine und/oder endokrine Drüsen.

In manchen Ausführungsformen werden die Drüsen ausgewählt aus der Gruppe bestehend aus Ohrspeichel-, Unterkieferspeichel-, Unterzungenspeichel-, kleine Speichel- und Augen-Drüsen. Wie die Auflistung suggeriert, können die Drüsen einzeln oder in einer beliebigen Kombination ausgewählt werden. Überdies ist dem Fachmann bekannt, dass manche Drüsen ein- oder beidseitig vorliegen und dementsprechend ferner kombiniert werden können. Daher können die verschiedenen Drüsen auch beispielsweise asymmetrisch (kontralateral) ausgewählt werden, das heißt, es kann beispielsweise die rechte Ohrspeicheldrüse und die linke Unterkieferspeicheldrüse ausgewählt werden oder *vice versa.* In einer Ausführungsform kann das Botulinumtoxin Botulinumtoxin Typ A oder Typ B sein.

In einer besonders bevorzugten Ausführungsform wird das Botulinumtoxin Typ A hochdosiert, oder in anderen Worten als "Hochdosistherapie" verabreicht. Dabei werden insgesamt zwischen 100 und 300 Einheiten Botulinumtoxin Typ A appliziert, besonders bevorzugt 150 Einheiten Botulinumtoxin Typ A appliziert. In dieser Ausführungsform kann das Botulinumtoxin Typ A allein ("Monotherapie") oder in Kombination mit anderen Agenzien wie z.B. einem Anticholinergikum verabreicht werden. In einer besonders bevorzugten Ausführungsform wird Botulinumtoxin Typ A allein appliziert. In einer Ausführungsform werden zwischen ein und vier Drüsen injiziert, in einer bevorzugten Ausführungsform werden zwei Drüsen injiziert. Es werden zwischen 75 und 150 Einheiten, besonders bevorzugt 100 Einheiten in eine Ohrspeicheldrüse appliziert und zwischen 25 und 75 Einheiten, besonders bevorzugt 50 Einheiten in eine Unterkieferspeicheldrüse appliziert. Besonders bevorzugt wird das hochdosierte Botulinumtoxin Typ A asymmetrisch (kontralateral) appliziert. Das Botulinumtoxin Typ A wurde asymmetrisch (100 Einheiten) in die rechte Ohrspeicheldrüse und in die kontralaterale, linke Unterkieferspeicheldrüsen (50 Einheiten) appliziert. Der Vorteil dieser Applikation liegt darin, dass zwei der vier großen Speicheldrüsen ohne Nebenwirkung maximal geschützt werden und der Patient zunächst noch während der initialen Radioliganden-Verabreichung durch die beiden übrigen Drüsen über eine ausreichende Speichelproduktion verfügt, bis zum Zeitpunkt der irreversiblen Beschädigung der beiden unbehandelten Drüsen, was häufig erst vollständig mit der zweiten oder nachfolgenden Radioliganden-Verabreichung passiert. Eine Hochdosistherapie aller vier Drüsen scheidet jedoch wegen des Risikos von Nebenwirkungen in Form von Schluckstörungen aus.

Diese ein- oder beidseitige Auswahl und Kombination der Speicheldrüsen gestaltet sich insbesondere für co- und multimorbide Patienten, Patienten mit einem Lebensalter von >75 Jahren, Patienten, die unter einer leichten kognitiven Störung ("mild cognitive impairment", MCI) oder Demenz leiden, oder auch Patienten mit einer reduzierten Lebenserwartung als vorteilhaft. Diese Patientengruppen sind frei kombinierbar und permutierbar, beispielsweise kann ein Patient >75 Jahre alt sein, unter einer leichten kognitiven Störung leiden und aufgrund einer Erkrankung eine Diagnostik oder Therapie mit einem Radioliganden erfordern. Auch ist hervorzuheben, dass in diesen Patientengruppen idealerweise auf ein Anticholinergikum verzichtet wird, um anticholinerge Nebenwirkungen zu vermeiden. Eine reduzierte Lebenserwartung im Sinne der Erfindung beträgt zwischen sechs und achtzehn Monaten, bevorzugterweise sechs Monate. Für diese Patientengruppe gestaltet sich eine Therapie mit weniger Injektionen, aber einer höheren Dosis als besonders vorteilhaft. Auch können durch die ein- oder beidseitige Auswahl und Kombination der Speicheldrüsen etwaige Nebenwirkungen sämtlicher Patienten vermieden werden.

In manchen Ausführungsformen ist die Drüse eine Unterkieferspeicheldrüse.

In manchen Ausführungsformen ist die Drüse eine seromuköse Drüse. In manchen Ausführungsformen ist die Drüse eine muköse Drüse. In manchen Ausführungsformen ist die Drüse eine seröse Drüse.

In manchen Ausführungsformen wirkt das Botulinumtoxin Typ A durch die Herabregulierung der α-1-Adrenorezeptoren in den Drüsen. In manchen Ausführungsformen wirkt das Botulinumtoxin auch durch die Herabregulierung der α-1-Adrenorezeptoren in den Drüsen, während andere Mechanismen ebenso wirken.

In manchen Ausführungsformen wird die Verwendung bevorzugt in einem Patientenkollektiv durchgeführt, das sich bereits einer Anti-Androgentherapie unterzogen hat. In manchen Ausführungsformen wird die Verwendung in einem Patientenkollektiv durchgeführt, das sich keiner Anti-Androgentherapie unterzogen hat. In weiteren Ausführungsformen wird die Verwendung in einem Patientenkollektiv durchgeführt, das sich während der Radioligand-Diagnostik oder -Therapie sich einer Anti-Androgentherapie unterzieht.

In manchen Ausführungsformen bewirkt Botulinumtoxin Typ A die Herabregulierung der Aquaporin-vermittelten Radionuklidaufnahme in der Ohrspeicheldrüse und Unterkieferspeicheldrüse.

### Beispiele:

Die vorliegende Erfindung wird durch die nachfolgenden, nicht einschränkenden Beispiele, detailliert beschrieben:

### Beispiel 1: Prävention von Strahlenschäden durch die Verwendung von Botulinumtoxin Typ A und einem Anticholinergikum bei der diagnostischen Verwendung von Radioliganden am Beispiel von ⁶⁸Ga-PSMA

Zwei Wochen vor der diagnostischen Verwendung von ⁶⁸Ga-PSMA in einem bildgebenden Verfahren werden insgesamt zwischen 20 und 900 Einheiten Botulinumtoxin Typ A, am Beispiel von Xeomin^{®} oder BOTOX^{®} 150 Einheiten, in Abhängigkeit des spezifischen Botulinumtoxin Typ A-Präparats, in die gewünschten Drüsen, wie die Unterkieferspeicheldrüse ein- oder beidseits und/oder ein- oder beidseits in die Ohrspeicheldrüsen appliziert. Drei Tage vor dem bildgebenden Verfahren unter der Verwendung von ⁶⁸Ga-PSMA wird die zusätzliche Verabreichung des Anticholinergikums initiiert. Das Anticholinergikum, hier Scopolamin, wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem diagnostischen Verfahren, ein Tag vor dem diagnostischen Verfahren, am Tag des diagnostischen Verfahrens) verabreicht, sowie bis 21 Tage nach dem diagnostischen Verfahren. Dies schützt zusätzlich die Unterzungenspeicheldrüse, die kleinen Speicheldrüsen und die Augendrüsen. Nach zwei Wochen (Tag 0) findet das bildgebende Verfahren mit ⁶⁸Ga-PSMA statt, um beispielsweise eine Bildgebung zur Verlaufskontrolle in einem Prostatakarzinompatienten zu erhalten. Die Aufnahme von ⁶⁸Ga-PSMA in den mit Botulinumtoxin behandelten Drüsen ist signifikant reduziert im Gegensatz zu beispielsweise Gehirn-Parenchym. Durch diese verminderte Aufnahme werden die jeweiligen Drüsen geschützt und funktionieren normal, daher werden persistierende Nebenwirkungen wie Mundtrockenheit, Augentrockenheit und ähnliche Symptome vermieden.

### Beispiel 2: Prävention von Strahlenschäden durch die Verwendung von Botulinumtoxin Typ B und einem Anticholinergikum bei der diagnostischen Verwendung von Radioliganden am Beispiel von ⁶⁸Ga-PSMA

Zwei Wochen vor der diagnostischen Verwendung von ⁶⁸Ga-PSMA in einem bildgebenden Verfahren werden insgesamt 3.000 Einheiten Botulinumtoxin Typ B, beispielsweise MYOBLOC^{®}, in die gewünschten Drüsen, wie die Unterkieferspeicheldrüse ein- oder beidseits und/oder ein- oder beidseits in die Ohrspeicheldrüsen appliziert. Drei Tage vor dem bildgebenden Verfahren unter der Verwendung von ⁶⁸Ga-PSMA wird die zusätzliche Verabreichung des Anticholinergikums initiiert. Das Anticholinergikum, hier Scopolamin, wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem diagnostischen Verfahren, ein Tag vor dem diagnostischen Verfahren, am Tag des diagnostischen Verfahrens) verabreicht, sowie 14 Tage nach dem diagnostischen Verfahren. Dies schützt zusätzlich die Unterzungenspeicheldrüse, die kleinen Speicheldrüsen und die Augendrüsen. Nach zwei Wochen (Tag 0) findet das bildgebende Verfahren mit ⁶⁸Ga-PSMA statt, um beispielsweise eine Bildgebung zur Verlaufskontrolle in einem Prostatakarzinompatienten zu erhalten. Die Aufnahme von ⁶⁸Ga-PSMA in den mit Botulinumtoxin behandelten Drüsen ist signifikant reduziert im Gegensatz zu beispielsweise Gehirn-Parenchym. Durch diese verminderte Aufnahme werden die jeweiligen Drüsen geschützt und funktionieren normal, daher werden persistierende Nebenwirkungen wie Mundtrockenheit, Augentrockenheit und ähnliche Symptome vermieden.

### Beispiel 3: Prävention von Strahlenschäden durch die Verwendung von Botulinumtoxin und einem Anticholinergikum bei der diagnostischen Verwendung von Radioliganden am Beispiel von ¹⁸F-PSMA sowie Botulinumtoxin Typ A

Zwei Wochen vor der diagnostischen Verwendung von ¹⁸F-PSMA in einem bildgebenden Verfahren werden insgesamt zwischen 20 und 900 Einheiten Botulinumtoxin Typ A, am Beispiel von Xeomin^{®} oder BOTOX^{®} 150 Einheiten, in Abhängigkeit des spezifischen Botulinumtoxin Typ A-Präparats in die gewünschten Drüsen, wie die Unterkieferspeicheldrüse ein- oder beidseits und/oder ein- oder beidseits in die Ohrspeicheldrüsen appliziert. Drei Tage vor dem bildgebenden Verfahren unter der Verwendung von ¹⁸F-PSMA wird zusätzlich das Anticholinergikum initiiert. Das Anticholinergikum, hier Scopolamin wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem diagnostischen Verfahren, ein Tag vor dem diagnostischen Verfahren, am Tag des diagnostischen Verfahrens) verabreicht, sowie bis 21 Tage nach dem diagnostischen Verfahren. Dies schützt zusätzlich die Unterzungenspeicheldrüse, die kleinen Speicheldrüsen und die Augendrüsen. Nach zwei Wochen (Tag 0) findet das bildgebende Verfahren mit ¹⁸F-PSMA statt, um beispielsweise eine Bildgebung zur Verlaufskontrolle in einem Prostatakarzinompatienten zu erhalten. Die Aufnahme von ¹⁸F-PSMA in den behandelten Drüsen ist signifikant reduziert im Gegensatz zu beispielsweise dem Gehirn-Parenchym. Durch diese verminderte Aufnahme werden die jeweiligen Drüsen geschützt und funktionieren normal, daher werden Nebenwirkungen wie Mundtrockenheit, Augentrockenheit und ähnliche Symptome vermieden.

### Beispiel 4: Prävention von Strahlenschäden durch die Verwendung von Botulinumtoxin und einem Anticholinergikum bei der diagnostischen Verwendung von Radioliganden am Beispiel von ¹⁸F-PSMA sowie Botulinumtoxin Typ B

Zwei Wochen vor der diagnostischen Verwendung von ⁶⁸Ga-PSMA in einem bildgebenden Verfahren werden insgesamt 3.000 Einheiten Botulinumtoxin Typ B, beispielsweise MYOBLOC^{®}, in die gewünschten Drüsen, wie die Unterkieferspeicheldrüse ein- oder beidseits und/oder ein- oder beidseits in die Ohrspeicheldrüsen appliziert. Drei Tage vor dem bildgebenden Verfahren unter der Verwendung von ¹⁸F-PSMA wird zusätzlich das Anticholinergikum initiiert. Das Anticholinergikum, hier Scopolamin, wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem diagnostischen Verfahren, ein Tag vor dem diagnostischen Verfahren, am Tag des diagnostischen Verfahrens) verabreicht, sowie bis 21 Tage nach dem diagnostischen Verfahren. Dies schützt zusätzlich die Unterzungenspeicheldrüse, die kleinen Speicheldrüsen und die Augendrüsen. Nach zwei Wochen (Tag 0) findet das bildgebende Verfahren mit ¹⁸F-PSMA statt, um beispielsweise eine Bildgebung zur Verlaufskontrolle in einem Prostatakarzinompatienten zu erhalten. Die Aufnahme von ¹⁸F-PSMA in den behandelten Drüsen ist signifikant reduziert im Gegensatz zu beispielsweise dem Gehirn-Parenchym. Durch diese verminderte Aufnahme werden die jeweiligen Drüsen geschützt und funktionieren normal, daher werden Nebenwirkungen wie Mundtrockenheit, Augentrockenheit und ähnliche Symptome vermieden.

### Beispiel 5: Prävention von Strahlenschäden durch die Verwendung von Botulinumtoxin Typ A und einem Anticholinergikum bei der therapeutischen Verwendung von Radioliganden am Beispiel von ²²⁵Ac-PSMA-617

Zwei Wochen vor der erstmaligen therapeutischen Verwendung von ²²⁵Ac-PSMA-617 werden insgesamt zwischen 20 und 900 Einheiten Botulinumtoxin Typ A, am Beispiel von Xeomin^{®} oder BOTOX^{®} 150 Einheiten, in Abhängigkeit des spezifischen Botulinumtoxin Typ A-Präparats, in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ²²⁵Ac-PSMA-617 wird zusätzlich das Anticholinergikum initiiert. Das Anticholinergikum, hier Scopolamin wird in Form von einem bis zwei transdermalen Pflaster alle drei Tage appliziert. Dabei wird ca. 1 mg Scopolamin mit annähernd konstanter Rate in die systemische Zirkulation freigesetzt. Alternativ wird das Anticholinergikum oral jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Dies schützt zusätzlich die Unterzungenspeicheldrüse, die kleinen Speicheldrüsen und die Augendrüsen. Nach zwei Wochen (Tag 0) findet die erstmalige Gabe von ²²⁵Ac-PSMA-617 (100 kBq/kg) statt, um die Behandlung eines Prostatakarzinompatienten zu beginnen. Die Aufnahme von ²²⁵Ac-PSMA-617 in den behandelten Drüsen ist signifikant reduziert zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ²²⁵Ac-PSMA-617 (100 kBq/kg) statt. Im weiteren Verlauf der Therapie mit ²²⁵Ac-PSMA-617 wird zwei Wochen vor einem weiteren therapeutischen Verfahren insgesamt zwischen 20 und 900 Einheiten Botulinumtoxin Typ A, am Beispiel von Xeomin^{®} oder BOTOX^{®} zwischen 50 und 150 Einheiten, in Abhängigkeit des spezifischen Botulinumtoxin Typ A-Präparats und in Abhängigkeit des Ausmaßes der Wirkung der vorangegangenen Botulinumtoxin-Injektion, verabreicht. Drei Tage vor Therapie unter der Verwendung von ²²⁵Ac-PSMA-617 wird zusätzlich das Anticholinergikum initiiert. Dieses wird jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Parallel dazu finden die Therapiezyklen mit ²²⁵Ac-PSMA-617 statt, die alle acht Wochen stattfinden. Voraussichtlich werden etwa vier Therapiezyklen mit ²²⁵Ac-PSMA-617 benötigt.

### Beispiel 6: Prävention von Strahlenschäden durch die Verwendung von Botulinumtoxin Typ B und einem Anticholinergikum bei der therapeutischen Verwendung von Radioliganden am Beispiel von ²²⁵Ac-PSMA-617

Zwei Wochen vor der erstmaligen therapeutischen Verwendung von ²²⁵Ac-PSMA-617 werden insgesamt 3.000 Einheiten Botulinumtoxin Typ B, beispielsweise MYOBLOC^{®}, in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ²²⁵Ac-PSMA-617 wird zusätzlich das Anticholinergikum initiiert. Das Anticholinergikum, hier Scopolamin, wird in Form von einem bis zwei transdermalen Pflaster alle drei Tage appliziert. Dabei wird ca. 1 mg Scopolamin mit annähernd konstanter Rate in die systemische Zirkulation freigesetzt. Alternativ wird das Anticholinergikum oral jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Dies schützt zusätzlich die Unterzungenspeicheldrüse, die kleinen Speicheldrüsen und die Augendrüsen. Nach zwei Wochen (Tag 0) findet die erstmalige Gabe von ²²⁵Ac-PSMA-617 (100 kBq/kg) statt, um die Behandlung eines Prostatakarzinompatienten zu beginnen. Die Aufnahme von ²²⁵Ac-PSMA-617 in den behandelten Drüsen ist signifikant reduziert zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ²²⁵Ac-PSMA-617 (100 kBq/kg) statt. Im weiteren Verlauf der Therapie mit ²²⁵Ac-PSMA-617 wird zwei Wochen vor dem therapeutischen Verfahren insgesamt zwischen 1000 und 3000 Einheiten Botulinumtoxin Typ B, in Abhängigkeit des Ausmaßes der Wirkung der vorangegangenen Botulinumtoxin-Injektion, in die gewünschten Drüsen verabreicht. Drei Tage vor Therapie unter der Verwendung von ²²⁵Ac-PSMA-617 wird zusätzlich das Anticholinergikum initiiert. Dieses wird jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Parallel dazu finden die Therapiezyklen mit ²²⁵Ac-PSMA-617 statt, die alle acht Wochen stattfinden. Voraussichtlich werden etwa vier Therapiezyklen mit ²²⁵Ac-PSMA-617 benötigt.

### Beispiel 7: Prävention von Strahlenschäden durch die Verwendung von Botulinumtoxin Typ A und einem Anticholinergikum bei der therapeutischen Verwendung von Radioliganden am Beispiel von ¹⁷⁷Lu-PSMA

Insgesamt zwischen 20 und 900 Einheiten Botulinumtoxin Typ A, am Beispiel von Xeomin^{®} oder BOTOX^{®} 150 Einheiten, in Abhängigkeit des spezifischen Botulinumtoxin Typ A-Präparats, wird in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ¹⁷⁷Lu-PSMA wird zusätzlich das Anticholinergikum verabreicht. Das Anticholinergikum, hier Scopolamin wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Dies schützt zusätzlich die Unterzungenspeicheldrüse, die kleinen Speicheldrüsen und die Augendrüsen. Nach zwei Wochen (Tag 0) findet die erstmalige Verabreichung von ¹⁷⁷Lu-PSMA (6 GBq/kg) statt, um die Behandlung eines Prostatakarzinompatienten zu beginnen. Die Aufnahme von ¹⁷⁷Lu-PSMA in den behandelten Drüsen ist signifikant reduziert im Gegensatz zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ¹⁷⁷Lu-PSMA (100 kBq/kg) statt. Im weiteren Verlauf der Therapie mit ¹⁷⁷Lu-PSMA wird ein Tag bis acht Wochen vor dem therapeutischen Verfahren insgesamt zwischen 20 und 900 Einheiten Botulinumtoxin Typ A, am Beispiel von Xeomin^{®} oder BOTOX^{®} zwischen 50 und 150 Einheiten, in Abhängigkeit des spezifischen Botulinumtoxin Typ A-Präparats, und in Abhängigkeit des Ausmaßes der Wirkung der vorangegangenen Botulinumtoxin-Injektion, in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ¹⁷⁷Lu-PSMA wird zusätzlich das Anticholinergikum verabreicht. Dieses wird jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Parallel dazu finden die Therapiezyklen mit ¹⁷⁷Lu-PSMA statt, die alle acht Wochen stattfinden. Voraussichtlich werden etwa vier Therapiezyklen mit ¹⁷⁷Lu-PSMA benötigt.

### Beispiel 8: Prävention von Strahlenschäden durch die Verwendung von Botulinumtoxin Typ B und einem Anticholinergikum bei der therapeutischen Verwendung von Radioliganden am Beispiel von ¹⁷⁷Lu-PSMA

Zwei Wochen vor der erstmaligen therapeutischen Verwendung von ¹⁷⁷Lu-PSMA werden insgesamt 3.000 Einheiten Botulinumtoxin Typ B, beispielsweise MYOBLOC^{®}, in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ¹⁷⁷Lu-PSMA wird zusätzlich das Anticholinergikum verabreicht. Das Anticholinergikum, hier Scopolamin wird in Form von einem bis zwei transdermalen Pflaster alle drei Tage appliziert. Dabei wird ca. 1 mg Scopolamin mit annähernd konstanter Rate innerhalb von 72 Stunden in die systemische Zirkulation freigesetzt. Alternativ wird das Anticholinergikum oral jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Dies schützt zusätzlich die Unterzungenspeicheldrüse, die kleinen Speicheldrüsen und die Augendrüsen. Nach zwei Wochen (Tag 0) findet die erstmalige Verabreichung von ¹⁷⁷Lu-PSMA (6 GBq/kg) statt, um die Behandlung eines Prostatakarzinompatienten zu beginnen. Die Aufnahme von ¹⁷⁷Lu-PSMA in den behandelten Drüsen ist signifikant reduziert im Gegensatz zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ¹⁷⁷Lu-PSMA (100 kBq/kg) statt. Im weiteren Verlauf der Therapie mit ¹⁷⁷Lu-PSMA werden zwei Wochen vor dem therapeutischen Verfahren insgesamt zwischen 1000 und 3000 Einheiten Botulinumtoxin Typ B, in Abhängigkeit des Ausmaßes der Wirkung der vorangegangenen Botulinumtoxin-Injektion, in die gewünschten Drüsen, wie die Unterkieferspeicheldrüse ein- oder beidseits und/oder ein- oder beidseits in die Ohrspeicheldrüsen appliziert. Drei Tage vor Therapie unter der Verwendung von ¹⁷⁷Lu-PSMA wird zusätzlich das Anticholinergikum verabreicht. Dieses wird jeden Tag (zwei Tage vor dem therapeutischen Verfahren, ein Tag vor dem therapeutischen Verfahren, am Tag des therapeutischen Verfahrens) verabreicht, sowie bis 21 Tage nach dem therapeutischen Verfahren. Parallel dazu finden die Therapiezyklen mit ¹⁷⁷Lu-PSMA statt, die alle acht Wochen stattfinden. Voraussichtlich werden etwa vier Therapiezyklen mit ¹⁷⁷Lu-PSMA benötigt.

### Beispiel 9: Prävention von Strahlenschäden durch die Verwendung von Botulinumtoxin Typ A bei der diagnostischen Verwendung von Radioliganden am Beispiel von ⁶⁸Ga-PSMA

Zwei Wochen vor der diagnostischen Verwendung von ⁶⁸Ga-PSMA in einem bildgebenden Verfahren werden insgesamt zwischen 20 und 900 Einheiten Botulinumtoxin Typ A, am Beispiel von Xeomin^{®} oder BOTOX^{®} insgesamt 150 Einheiten, in Abhängigkeit des spezifischen Botulinumtoxin Typ A-Präparats, appliziert. Das Botulinumtoxin Typ A wird in einem fixen Dosisverhältnis 2/3 (Ohrspeicheldrüse) zu 1/3 (Unterkieferspeicheldrüse) appliziert, das heißt, in jede der beiden Ohrspeicheldrüsen werden 50 Einheiten Botulinumtoxin Typ A appliziert und in jede der beiden Unterkieferspeicheldrüsen werden 25 Einheiten Botulinumtoxin Typ A appliziert. Nach zwei Wochen (Tag 0) findet das bildgebende Verfahren mit ⁶⁸Ga-PSMA statt, um beispielsweise eine Bildgebung zur Verlaufskontrolle in einem Prostatakarzinompatienten zu erhalten. Die Aufnahme von ⁶⁸Ga-PSMA in den mit Botulinumtoxin behandelten Drüsen ist signifikant reduziert im Gegensatz zu beispielsweise Gehirn-Parenchym. Durch diese verminderte Aufnahme werden die jeweiligen Drüsen geschützt und funktionieren normal, daher werden persistierende Nebenwirkungen wie Mundtrockenheit, Augentrockenheit und ähnliche Symptome vermieden.

### Beispiel 10: Prävention von Strahlenschäden durch die Verwendung von Botulinumtoxin Typ B bei der diagnostischen Verwendung von Radioliganden am Beispiel von ⁶⁸Ga-PSMA

Zwei Wochen vor der diagnostischen Verwendung von ⁶⁸Ga-PSMA in einem bildgebenden Verfahren werden insgesamt 3.000 Einheiten Botulinumtoxin Typ B, beispielsweise MYOBLOC^{®}, appliziert. Das Botulinumtoxin Typ B wird in einem fixen Dosisverhältnis 2/3 (Ohrspeicheldrüse) zu 1/3 (Unterkieferspeicheldrüse) appliziert. Bei einer Annahme von 3.000 Einheiten insgesamt bedeutet dies, dass in jede der beiden Ohrspeicheldrüsen 1000 Einheiten Botulinumtoxin Typ B appliziert werden und in jede der beiden Unterkieferspeicheldrüsen 500 Einheiten Botulinumtoxin Typ B appliziert werden. Nach zwei Wochen (Tag 0) findet das bildgebende Verfahren mit ⁶⁸Ga-PSMA statt, um beispielsweise eine Bildgebung zur Verlaufskontrolle in einem Prostatakarzinompatienten zu erhalten. Die Aufnahme von ⁶⁸Ga-PSMA in den mit Botulinumtoxin behandelten Drüsen ist signifikant reduziert im Gegensatz zu beispielsweise Gehirn-Parenchym. Durch diese verminderte Aufnahme werden die jeweiligen Drüsen geschützt und funktionieren normal, daher werden persistierende Nebenwirkungen wie Mundtrockenheit, Augentrockenheit und ähnliche Symptome vermieden.

### Beispiel 11: Prävention von Strahlenschäden durch die Verwendung von Botulinumtoxin Typ A bei der therapeutischen Verwendung von Radioliganden am Beispiel von ²²⁵Ac-PSMA-617

Zwei Wochen vor der therapeutischen Verwendung von ²²⁵Ac-PSMA-617 in einem bildgebenden Verfahren werden insgesamt zwischen 20 und 900 Einheiten Botulinumtoxin Typ A, am Beispiel von Xeomin^{®} oder BOTOX^{®} 150 Einheiten, in Abhängigkeit des spezifischen Botulinumtoxin Typ A-Präparats, appliziert. Das Botulinumtoxin Typ A (Xeomin^{®} oder BOTOX^{®}) wird in einem fixen Dosisverhältnis 2/3 (Ohrspeicheldrüse) zu 1/3 (Unterkieferspeicheldrüse) appliziert, das heißt, in jede der beiden Ohrspeicheldrüsen werden 50 Einheiten Botulinumtoxin Typ A appliziert und in jede der beiden Unterkieferspeicheldrüsen werden 25 Einheiten Botulinumtoxin Typ A appliziert. Nach zwei Wochen (Tag 0) findet die erstmalige Gabe von ²²⁵Ac-PSMA-617 (100 kBq/kg) statt, um die Behandlung eines Prostatakarzinompatienten zu beginnen. Die Aufnahme von ²²⁵Ac-PSMA-617 in den behandelten Drüsen ist signifikant reduziert zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ²²⁵Ac-PSMA-617 (100 kBq/kg) statt. Im weiteren Verlauf der Therapie mit ²²⁵Ac-PSMA-617 wird zwei Wochen vor dem therapeutischen Verfahren insgesamt 20 bis 900 Einheiten Botulinumtoxin Typ A, am Beispiel von Xeomin^{®} oder BOTOX^{®} insgesamt 50 bis 150 Einheiten, in Abhängigkeit des Ausmaßes der Wirkung der vorangegangenen Botulinumtoxin-Injektion, verabreicht. Das Botulinumtoxin Typ A (Xeomin^{®} oder BOTOX^{®}) wird in einem fixen Dosisverhältnis 2/3 (Ohrspeicheldrüse) zu 1/3 (Unterkieferspeicheldrüse) verabreicht. Parallel dazu finden die Therapiezyklen mit ²²⁵Ac-PSMA-617 statt, die alle acht Wochen stattfinden. Voraussichtlich werden etwa vier Therapiezyklen mit ²²⁵Ac-PSMA-617 benötigt.

### Beispiel 12: Prävention von Strahlenschäden durch die Verwendung von Botulinumtoxin Typ B bei der therapeutischen Verwendung von Radioliganden am Beispiel von ²²⁵Ac-PSMA-617

Zwei Wochen vor der therapeutischen Verwendung von ²²⁵Ac-PSMA-617 in einem bildgebenden Verfahren werden insgesamt zwischen 3.000 Einheiten Botulinumtoxin Typ B, beispielsweise MYOBLOC^{®}, appliziert. Das Botulinumtoxin Typ B wird in einem fixen Dosisverhältnis 2/3 (Ohrspeicheldrüse) zu 1/3 (Unterkieferspeicheldrüse) appliziert. Bei einer Annahme von 3.000 Einheiten insgesamt bedeutet dies, dass in jede der beiden Ohrspeicheldrüsen 1000 Einheiten Botulinumtoxin Typ B verabreicht werden und in jede der beiden Unterkieferspeicheldrüsen 500 Einheiten Botulinumtoxin Typ B verabreicht werden. Nach zwei Wochen (Tag 0) findet die erstmalige Gabe von ²²⁵Ac-PSMA-617 (100 kBq/kg) statt, um die Behandlung eines Prostatakarzinompatienten zu beginnen. Die Aufnahme von ²²⁵Ac-PSMA-617 in den behandelten Drüsen ist signifikant reduziert zu beispielsweise dem Gehirn-Parenchym. Durch diese verminderte Aufnahme werden die jeweiligen Drüsen geschützt und funktionieren normal, daher werden schwere, persistierende und irreversible Nebenwirkungen wie beispielsweise die stark die Lebensqualität einschränkende Austrocknung der Augen und chronische schwere Entzündungen von Augen und Lidrändern vermieden, es werden Schluckstörungen, damit verbundener Gewichtsverlust, Entzündungen von Mundschleimhaut und Zahnschäden und ähnliche Symptome verhindert. Nach etwa acht Wochen findet eine weitere Gabe von ²²⁵Ac-PSMA-617 (100 kBq/kg) statt. Im weiteren Verlauf der Therapie mit ²²⁵Ac-PSMA-617 wird zwei Wochen vor dem therapeutischen Verfahren insgesamt zwischen 1000 und 3000 Einheiten Botulinumtoxin Typ B, in Abhängigkeit des Ausmaßes der Wirkung der vorangegangenen Botulinumtoxin-Injektion, verabreicht. Parallel dazu finden die Therapiezyklen mit ²²⁵Ac-PSMA-617 statt, die alle acht Wochen stattfinden. Voraussichtlich werden etwa vier Therapiezyklen mit ²²⁵Ac-PSMA-617 benötigt.

### Beispiel 13: Prävention von Strahlenschäden durch die Verwendung von Botulinumtoxin Typ A und einem Anticholinergikum bei der therapeutischen Verwendung von ¹⁷⁷Lu-PSMA-617

Zwei Wochen (14 Tage) vor der therapeutischen Verwendung von ¹⁷⁷Lu-PSMA-617 wurden insgesamt 120 Einheiten Botulinumtoxin Typ A (Xeomin^{®}) appliziert. Das Botulinumtoxin Typ A wurde beidseits (jeweils 40 Einheiten) in die Ohrspeicheldrüsen bzw. die Unterkieferspeicheldrüsen (jeweils 20 Einheiten) appliziert.

Drei Tage vor Therapie unter der Verwendung von ¹⁷⁷Lu-PSMA-617 wurde zusätzlich das Anticholinergikum verabreicht. Das Anticholinergikum, hier Scopolamin wird in Form von einem transdermalen Pflaster alle drei Tage appliziert. Dabei wird ca. 1 mg Scopolamin mit annähernd konstanter Rate innerhalb von 72 Stunden in die systemische Zirkulation freigesetzt. Jenes Scopolamin-Pflaster wurde an Tag 0 und Tag 3 der ¹⁷⁷Lu-PSMA-617-Therapie erneuert, so dass der Patient bis zu sechs Tagen nach Therapie zusätzlich mit Scopolamin behandelt wurde. Dies schützt zusätzlich die Unterzungenspeicheldrüse, die kleinen Speicheldrüsen und die Augendrüsen.

Unmittelbar vor der ersten ¹⁷⁷Lu-PSMA-617-Therapie /Tag 0 zeigte sich eine normale Speichelproduktion. In diesem Fall betrug der Speichel 5,7 Gramm im Saxon-Test, wobei der Normwert ≥ 2,75 Gramm Speichelproduktion nach zwei Minuten kauen beträgt. Nach zwei Wochen (Tag 0) fand die erstmalige Gabe von ¹⁷⁷Lu-PSMA-617 (6 GBq/kg) statt, um die Behandlung eines Prostatakarzinompatienten zu beginnen.

Nach etwa acht Wochen (Tag 56) fand eine weitere Gabe von ¹⁷⁷Lu-PSMA-617 (6 GBq/kg) statt. Nach Abschluss der Behandlung (zwei Therapiezyklen an Tag 0 und 56) und ca. acht Wochen Nachbeobachtungszeit zeigte sich an Tag 108 eine unverändert normale Speichelproduktion. Hier betrug der Speichel im Saxon-Test 5,5 Gramm, wobei der Normwert ≥ 2,75 Gramm Speichelproduktion nach zwei Minuten kauen beträgt. Schluckstörungen traten nicht auf. Augentrockenheit wurde nicht berichtet.

### Beispiel 14: Prävention von Strahlenschäden durch die Verwendung von Botulinumtoxin Typ A bei der therapeutischen Verwendung von ¹⁷⁷Lu-PSMA-617

Zweieinhalb Wochen (18 Tage) vor der therapeutischen Verwendung von ¹⁷⁷Lu-PSMA-617 wurden insgesamt 150 Einheiten Botulinumtoxin Typ A (Xeomin^{®}) appliziert. Das Botulinumtoxin Typ A wurde asymmetrisch (100 Einheiten) in die rechte Ohrspeicheldrüse und in die kontralaterale, linke Unterkieferspeicheldrüsen (50 Einheiten) appliziert.

Unmittelbar vor der ersten ¹⁷⁷Lu-PSMA-617-Therapie /Tag 0 zeigte sich eine normale Speichelproduktion. In diesem Fall betrug der Speichel im Saxon-Test 4,6 Gramm, wobei der Normwert ≥ 2,75 Gramm Speichelproduktion nach zwei Minuten kauen beträgt. Nach zweieinhalb Wochen (Tag 0) fand die erstmalige Gabe von ¹⁷⁷Lu-PSMA-617 (6 GBq/kg) statt, um die Behandlung eines Prostatakarzinompatienten zu beginnen.

Nach etwa acht Wochen (Tag 56) fand eine weitere Gabe von ¹⁷⁷Lu-PSMA-617 (6 GBq/kg) statt. Nach Abschluss der Behandlung (zwei Therapiezyklen an Tag 0 und 56) und ca. acht Wochen Nachbeobachtungszeit zeigte sich an Tag 116 eine unverändert normale Speichelproduktion. Hier betrug der Speichel im Saxon-Test 4,3 Gramm, wobei der Normwert ≥ 2,75 Gramm Speichelproduktion nach zwei Minuten kauen beträgt. Schluckstörungen traten nicht auf. Augentrockenheit wurde nicht berichtet.

### Beispiel 15: Prävention von Strahlenschäden durch die Verwendung von Botulinumtoxin Typ B bei der therapeutischen Verwendung von ¹⁷⁷Lu-PSMA-617

Zwei Wochen (13 Tage) vor der therapeutischen Verwendung von ¹⁷⁷Lu-PSMA-617 wurden insgesamt 6000 Einheiten Botulinumtoxin Typ B (Neurobloc^{®}) appliziert. Das Botulinumtoxin Typ B wurde asymmetrisch (4000 Einheiten) in die rechte Ohrspeicheldrüse und in die kontralaterale, linke Unterkieferspeicheldrüsen (2000 Einheiten) appliziert.

Unmittelbar vor der ersten ¹⁷⁷Lu-PSMA-617-Therapie /Tag 0 zeigte sich eine normale Speichelproduktion. Nach zweieinhalb Wochen (Tag 0) fand die erstmalige Gabe von ¹⁷⁷Lu-PSMA-617 (6 GBq/kg) statt, um die Behandlung eines Prostatakarzinompatienten zu beginnen.

Nach etwa acht Wochen (Tag 56) fand eine weitere Gabe von ¹⁷⁷Lu-PSMA-617 (6 GBq/kg) statt. Nach Abschluss der Behandlung (zwei Therapiezyklen an Tag 0 und 56) und ca. acht Wochen Nachbeobachtungszeit zeigte sich an Tag 116 eine unverändert normale Speichelproduktion. Schluckstörungen traten nicht auf. Augentrockenheit wurde nicht berichtet.

Auf Basis der Beispiele 13-15 sind die folgenden weiteren Behandlungen der vier folgenden Patientengruppen geplant:
1) Patienten im mittleren Lebensalter 50 - 75 Jahre
2) Patienten im höheren Lebensalter > 75 Jahre
3) Multimorbide Patienten und/oder Patienten mit mild cognitive impairment (MCI)
4) Patienten mit zu erwartender kurzer Überlebenszeit < 1,5 Jahre

### Beispiel 16: Geplante Behandlung von Patienten der Patientengruppe 1 (mittleres Lebensalter 50-75 Jahre): Prävention von Strahlenschäden durch die Verwendung von Botulinumtoxin Typ A und einem Anticholinergikum bei der therapeutischen Verwendung von ²²⁵Ac-PSMA-617

Drei Wochen (21 Tage) vor der therapeutischen Verwendung von ²²⁵Ac-PSMA-617 werden insgesamt 150 Einheiten Botulinum Toxin Typ A (Xeomin^{®}) appliziert. Das Botulinumtoxin Typ A wird beidseits (jeweils 50 Einheiten) in die Ohrspeicheldrüsen bzw. die Unterkieferspeicheldrüsen (jeweils 25 Einheiten) appliziert (d.h im Verhältnis 2/3 Ohrspeicheldrüse zu 1/3 Unterkieferspeicheldrüse).

Drei Tage vor Therapie unter der Verwendung von ²²⁵Ac-PSMA-617 wird zusätzlich das Anticholinergikum verabreicht. Das Anticholinergikum, hier Scopolamin wird in Form von einem transdermalen Pflaster alle drei Tage appliziert. Dabei wird ca. 1 mg Scopolamin mit annähernd konstanter Rate innerhalb von 72 Stunden in die systemische Zirkulation freigesetzt. Jenes Scopolamin-Pflaster wird an Tag 0 und Tag 3 und Tag 6 der ²²⁵Ac-PSMA-617 - Therapie erneuert, so dass der Patient bis neun Tagen nach Therapie zusätzlich mit Scopolamin behandelt wird. Dies schützt zusätzlich die Unterzungenspeicheldrüse, die kleinen Speicheldrüsen und die Augendrüsen

### Beispiel 17: Geplante Behandlung von Patienten der Patientengruppe 2 (höheres Lebensalter >75 Jahre): Prävention von Strahlenschäden durch die Verwendung von Botulinumtoxin Typ B bei der therapeutischen Verwendung von ²²⁵Ac-PSMA-617

Zwei Wochen (14 Tage) vor der therapeutischen Verwendung von ²²⁵Ac-PSMA-617 werden insgesamt 6000 Einheiten Botulinum Toxin Typ B (Neurobloc^{®}) appliziert. Das Botulinumtoxin Typ B wird beidseits (jeweils 2000 Einheiten) in die Ohrspeicheldrüsen bzw. die Unterkieferspeicheldrüsen (jeweils 1000 Einheiten) appliziert (d.h im Verhältnis 2/3 Ohrspeicheldrüse zu 1/3 Unterkieferspeicheldrüse.

### Beispiel 18: Geplante Behandlung von Patienten der Patientengruppen 3+4 (multimorbide Patienten, / Patienten mit MCI / Patienten mit reduzierter Lebenserwartung): Prävention von Strahlenschäden durch die Verwendung von Botulinumtoxin Typ A bei der therapeutischen Verwendung von ²²⁵Ac-PSMA-617

Drei Wochen (21 Tage) vor der therapeutischen Verwendung von ²²⁵Ac-PSMA-617 werden insgesamt 150 Einheiten Botulinum Toxin Typ A (Xeomin^{®}) appliziert. Das Botulinumtoxin Typ A wird asymmetrisch einseitig als Hochdosismonotherapie (100 Einheiten) in die Ohrspeicheldrüse rechts bzw. einseitig in die Unterkieferspeicheldrüse links (50 Einheiten) appliziert (d.h. im Verhältnis 2/3 Ohrspeicheldrüse zu 1/3 Unterkieferspeicheldrüse.)

Der Begriff der Hochdosismonotherapie mit Botulinum Toxin A (Xeomin^{®}) bezieht sich auf die jeweilige Einzeldosis in den beiden Drüsen, die in einem bisher nicht publizierten Dosisbereich pro Drüse liegt, aber gut verträglich ist, wie sich aus den bereits behandelten Patienten zeigt. Die drüsenbezogene Hochdosismonotherapie darf bei dieser Patientengruppe keinesfalls für alle vier Drüsen appliziert werden, um Schluckstörungen zu vermeiden. Die asymmetrische Monotherapie mit Botulinum Toxin Typ A in jeweils nur eine kontralaterale Ohr- und Unterkieferspeicheldrüse dient ebenfalls der Vermeidung von Anticholinergikaassoziierten Nebenwirkungen bei Patientengruppe 3. Die beiden zunächst unverändert Speichel produzierenden, nicht-injizierten Drüsen verkürzen (bis zu deren vollständiger Schädigung bei der zweiten oder dritten oder vierten ²²⁵Ac-PSMA-617 Behandlung) die Zeit der Mundtrockenheit während des Therapiezeitraums um den Preis der vollständigen Zerstörung von zwei der vier Speicheldrüsen durch wiederholte ²²⁵Ac-PSMA-617 Behandlungen und ist daher aus Aspekten der Lebensqualität besonders für die Patientengruppe 4 mit einer zu erwartenden kurzen Überlebenszeit geeignet.

## Patentansprüche

1. Botulinumtoxin in Kombination mit einem Anticholinergikum zur Verwendung in einem Verfahren zur Prävention von Strahlenschäden der Drüsen, wobei die Strahlenschäden von Radioliganden hervorgerufen werden und wobei das Anticholinergikum aus der Gruppe bestehend aus Tropicamid, Atropin, Scopolamin, Glycopyrrolat, Amitriptylin, Clonidin, Ipratropium-Bromid und Trihexyphenidyl ausgewählt wird.

2. Botulinumtoxin in Kombination mit einem Anticholinergikum zur Verwendung in einem Verfahren zur Prävention von Strahlenschäden der Drüsen gemäß Anspruch 1, wobei die Strahlenschäden von einem PSMA-Radioliganden hervorgerufen werden.

3. Botulinumtoxin in Kombination mit einem Anticholinergikum zur Verwendung in einem Verfahren zur Prävention von Strahlenschäden der Drüsen gemäß Anspruch 1 und 2, wobei die Strahlenschäden ausgewählt aus der Gruppe von ²²⁵Ac-PSMA, ⁶⁸Ga-PSMA, ¹⁸F-PSMA oder ¹⁷⁷Lu-PSMA, hervorgerufen werden.

4. Das Botulinumtoxin in Kombination mit einem Anticholinergikum zur Verwendung gemäß Anspruch 1, wobei das Botulinumtoxin ausgewählt wird aus der Gruppe bestehend aus den Botulinumtoxin-Typen A, B, C, D, E, F und G.

5. Das Botulinumtoxin in Kombination mit einem Anticholinergikum zur Verwendung gemäß einem der vorhergehenden Ansprüche, wobei das Botulinumtoxin zwischen 1 und 10.000 Einheiten umfasst.

6. Das Botulinumtoxin in Kombination mit einem Anticholinergikum zur Verwendung gemäß einem der vorhergehenden Ansprüche, wobei das Botulinumtoxin ein Botulinumtoxin Typ A ist.

7. Das Botulinumtoxin in Kombination mit einem Anticholinergikum zur Verwendung gemäß einem der vorhergehenden Ansprüche, das zwischen 1 und 1500 Einheiten an Botulinumtoxin Typ A umfasst.

8. Das Botulinumtoxin in Kombination mit einem Anticholinergikum zur Verwendung gemäß einem der vorhergehenden Ansprüche, wobei das Botulinumtoxin ein Botulinumtoxin Typ B ist.

9. Das Botulinumtoxin in Kombination mit einem Anticholinergikum zur Verwendung gemäß einem der vorhergehenden Ansprüche, das zwischen 100 und 10000 Einheiten an Botulinumtoxin Typ B umfasst.

10. Das Botulinumtoxin in Kombination mit einem Anticholinergikum zur Verwendung gemäß einem der vorhergehenden Ansprüche, wobei das Botulinumtoxin ein Botulinumtoxin Typ E oder Typ F ist.

11. Das Botulinumtoxin in Kombination mit einem Anticholinergikum zur Verwendung gemäß einem der vorhergehenden Ansprüche, das zwischen 1 und 10.000 Einheiten an Botulinumtoxin Typ E oder Typ F umfasst.

12. Das Botulinumtoxin in Kombination mit einem Anticholinergikum zur Verwendung gemäß einem der vorhergehenden Ansprüche, wobei das Anticholinergikum transdermal, oral oder intravenös zu verabreichen ist.

13. Das Botulinumtoxin in Kombination mit einem Anticholinergikum zur Verwendung gemäß einem der vorhergehenden Ansprüche, wobei das Anticholinergikum Scopolamin ist, wobei das Scopolamin transdermal zu verabreichen ist.

14. Botulinumtoxin Typ B zur Verwendung in einem Verfahren zur Prävention von Strahlenschäden der Ohrspeichel- und Unterkieferspeicheldrüsen, wobei die Strahlenschäden von Radioliganden hervorgerufen werden und wobei das Botulinumtoxin Typ B in die jeweilige Ohrspeicheldrüse und die kontralaterale Unterkieferspeicheldrüse zu verabreichen ist.

15. Botulinumtoxin Typ A zur Verwendung in einem Verfahren zur Prävention von Strahlenschäden der Drüsen, wobei die Strahlenschäden von Radioliganden hervorgerufen werden und wobei das Botulinumtoxin Typ A in die jeweilige Ohrspeicheldrüse und in die kontralaterale Unterkieferspeicheldrüse zu verabreichen ist.

## Claims

1. A botulinum toxin in combination with an anticholinergic for use in a method of preventing radiation damage to the glands, wherein the radiation damage is caused by radioligands and wherein the anticholinergic is selected from the group consisting of tropicamide, atropine, scopolamine, glycopyrrolate, amitriptyline, clonidine, ipratropium bromide and trihexyphenidyl.

2. The botulinum toxin in combination with an anticholinergic for use in a method of preventing radiation damage to the glands according to claim 1, wherein the radiation damage is caused by a PSMA radioligand.

3. The botulinum toxin in combination with an anticholinergic for use in a method of preventing radiation damage to the glands according to claims 1 and 2, wherein the radiation damage is caused by one selected from the group consisting of ²²⁵Ac-PSMA, ⁶⁸Ga-PSMA, ¹⁸F-PSMA or ¹⁷⁷Lu-PSMA.

4. The botulinum toxin in combination with an anticholinergic for use according to claim 1, wherein the botulinum toxin is selected from the group consisting of botulinum toxin types A, B, C, D, E, F and G.

5. The botulinum toxin in combination with an anticholinergic for use according to any one of the preceding claims, wherein the botulinum toxin comprises between 1 and 10,000 units.

6. The botulinum toxin in combination with an anticholinergic for use according to any one of the preceding claims, wherein the botulinum toxin is a botulinum toxin type A.

7. The botulinum toxin in combination with an anticholinergic for use according to any one of the preceding claims comprising between 1 and 1,500 units of botulinum toxin type A.

8. The botulinum toxin in combination with an anticholinergic for use according to any one of the preceding claims, wherein the botulinum toxin is a botulinum toxin type B.

9. The botulinum toxin in combination with an anticholinergic for use according to any one of the preceding claims, comprising between 100 and 10,000 units of botulinum toxin type B.

10. The botulinum toxin in combination with an anticholinergic for use according to any one of the preceding claims, wherein the botulinum toxin is a botulinum toxin type E or type F.

11. The botulinum toxin in combination with an anticholinergic for use according to any one of the preceding claims comprising between 1 and 10,000 units of botulinum toxin type E or type F.

12. The botulinum toxin in combination with an anticholinergic for use according to any one of the preceding claims, wherein the anticholinergic is to be administered transdermally, orally or intravenously.

13. The botulinum toxin in combination with an anticholinergic for use according to any one of the preceding claims, wherein the anticholinergic is scopolamine, wherein the scopolamine is to be administered transdermally.

14. A botulinum toxin type B for use in a method for preventing radiation damage to the parotid, and submandibular glands, wherein radiation damage is caused by radioligands and wherein the botulinum toxin type B is to be administered into the respective parotid gland and the contralateral submandibular gland.

15. A botulinum toxin type A for use in a method for preventing radiation damage to the glands, wherein radiation damage is caused by radioligands and wherein the botulinum toxin type A is to be administered into the respective parotid gland and contralateral submandibular gland.

## Revendications

1. Toxine botulique combinée à un anticholinergique pour l'utilisation dans un procédé de prévention de lésions des glandes causées par les rayons, les lésions causées par les rayons étant provoquées par des radioligands et l'anticholinergique étant choisi dans le groupe consistant en le tropicamide, l'atropine, la scopolamine, le glycopyrrolate, l'amitriptyline, la clonidine, le bromure d'ipratropium et le trihexyphénidyle.

2. Toxine botulique combinée à un anticholinergique pour l'utilisation dans un procédé de prévention de lésions des glandes causées par les rayons selon la revendication 1, les lésions causées par les rayons étant provoquées par un PSMA-radioligand.

3. Toxine botulique combinée à un anticholinergique pour l'utilisation dans un procédé de prévention de lésions des glandes causées par les rayons selon la revendication 1 et 2, les lésions causées par les rayons étant provoquées par des éléments choisis dans le groupe des ²²⁵Ac-PSMA, ⁶⁸Ga-PSMA, ¹⁸F-PSMA ou ¹⁷⁷Lu-PSMA.

4. Toxine botulique combinée à un anticholinergique pour l'utilisation selon la revendication 1, la toxine botulique étant choisie dans le groupe consistant en les types de toxine botulique A, B, C, D, E, F et G.

5. Toxine botulique combinée à un anticholinergique pour l'utilisation selon l'une des revendications précédentes, la toxine botulique comprenant entre 1et 10 000 unités.

6. Toxine botulique combinée à un anticholinergique pour l'utilisation selon l'une des revendications précédentes, la toxine botulique étant de type A.

7. Toxine botulique combinée à un anticholinergique pour l'utilisation selon l'une des revendications précédentes, la toxine botulique comprenant entre 1et 1 500 unités de toxine botulique de type A.

8. Toxine botulique combinée à un anticholinergique pour l'utilisation selon l'une des revendications précédentes, la toxine botulique étant de type B.

9. Toxine botulique combinée à un anticholinergique pour l'utilisation selon l'une des revendications précédentes, qui comprend entre 100 et 10 000 unités de toxine botulique de type B.

10. Toxine botulique combinée à un anticholinergique pour l'utilisation selon l'une des revendications précédentes, la toxine botulique étant une toxine botulique de type E ou de type F.

11. Toxine botulique combinée à un anticholinergique pour l'utilisation selon l'une des revendications précédentes, qui comprend entre 100 et 10 000 unités de toxine botulique de type E ou de type F.

12. Toxine botulique combinée à un anticholinergique pour l'utilisation selon l'une des revendications précédentes, l'anticholinergique devant être administré par voie transdermique, orale ou intraveineuse.

13. Toxine botulique combinée à un anticholinergique pour l'utilisation selon l'une des revendications précédentes, l'anticholinergique étant la scopolamine, la scopolamine devant être administrée par voie transdermique.

14. Toxine botulique de type B pour l'utilisation dans un procédé de prévention de lésions des glandes parotides et sous-mandibulaires causées par les rayons, les lésions causées par les rayons étant provoquées par des radioligands et la toxine botulique de type B devant être administrée dans la glande parotide respective et la glande sous-mandibulaire controlatérale.

15. Toxine botulique de type A pour l'utilisation dans un procédé de prévention de lésions des glandes causées par les rayons, les lésions causées par les rayons étant provoquées par des radioligands et la toxine botulique de type A devant être administrée dans la glande parotide respective et la glande sous-mandibulaire controlatérale.
